(19) Europäisches Patentamt
European Patent Office
Office européen des brevets

(11) EP 2 248 531 A1

(12) EUROPEAN PATENT APPLICATION

(43) Date of publication:
**10.11.2010 Bulletin 2010/45**

(51) Int Cl.:
***A61K 38/28*** *(2006.01)*

(21) Application number: **10006250.4**

(22) Date of filing: **03.08.2005**

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.08.2004 US 598246 P**

(62) Document number(s) of the earlier application(s) in accordance with Art. 76 EPC:
**05778823.4 / 1 773 376**

(71) Applicant: **Emisphere Technologies, Inc.**
**Tarrytown, New York 10591 (US)**

(72) Inventors:
- **Arbit, Ehud**
**Tarrytown, NY 10591 (US)**
- **Goldberg, Michael, M.**
**Tarrytown, NY 10591 (US)**
- **Dinh, Steven**
**Tarrytown, NY 10591 (US)**

(74) Representative: **Engelhard, Markus**
**Forrester & Boehmert**
**Pettenkoferstrasse 20-22**
**80336 München (DE)**

Remarks:
This application was filed on 16-06-2010 as a divisional application to the application mentioned under INID code 62.

# (54) Antidiabetic oral insulin-biguanide combination

(57) Pharmaceutical dosage forms, comprising insulin, a delivery agent that facilitates insulin transport in a therapeutically effective amount to the bloodstream and a biguanide, such as metformin, are disclosed for oral administration to a patient for the treatment of diabetes. Also disclosed are methods for achieving improved glucose tolerance and glycemic control in a diabetic mammal without any statistically significant increase in weight, risk of hypoglycemia or hyperinsulinemia, and the need for monitoring blood glucose concentrations or $HbA_1c$ levels, and methods for reducing the incidence and/or severity of one or more disease states associated with chronic dosing of insulin; for prophylactically sparing β-cell function or for preventing β-cell death or dysfunction in a mammal with impaired glucose tolerance or early stage diabetes mellitus; and for long-term protection from developing (or delaying the onset of) overt or insulin dependent diabetes in a mammal with impaired glucose tolerance or early stage diabetes.

EP 2 248 531 A1

## Description

### FIELD OF THE INVENTION

This invention relates to the oral delivery of an antidiabetic formulation comprising insulin and a biguanide, such as metformin, in a therapeutically effective amount to the bloodstream as part of a therapeutic regimen for the treatment of diabetes. The invention is also directed to therapies and protocols for administration of oral pharmaceutical dosage forms of an antidiabetic formulation comprising insulin and a biguanide, such as metformin, on a chronic basis to pre-diabetics, including those with impaired glucose tolerance and/or insulin resistance, to early stage diabetics, and to late stage diabetics.

### BACKGROUND OF THE INVENTION

The hormone insulin contributes to the normal regulation of blood glucose levels through its release by the pancreas, more specifically by the β-cells of a major type of pancreatic tissue (the islets of Langerhans), so that glucose can be used as a source of energy. Insulin secretion is a regulated process that, in normal subjects, provides stable concentrations of glucose in blood during both fasting and fed states. In healthy humans, insulin is secreted from the pancreas into the portal vein, which carries the insulin to the liver, and facilitates (and increases the rate of) glucose transport through the membranes of many cells of the body, particularly skeletal muscle and adipose tissue. Insulin has three basic effects: the enhanced rate of glucose metabolism, the promotion of increased glycogen stores in muscle and adipose tissue, and decreased circulating blood glucose concentration.

The liver utilizes and/or metabolizes a large portion of the insulin that it receives from the portal circulation and plays a key role in the metabolism of glucose. In the presence of excess insulin, excess glucose, or both, the liver modulates the production of glucose released into the blood; and, in the absence of insulin or when the blood glucose concentration falls very low, the liver manufactures glucose from glycogen and releases it into the blood. The liver acts as a key blood glucose buffer mechanism by keeping blood glucose concentrations from rising too high or from falling too low.

Blood glucose concentration is the principal stimulus to insulin secretion in healthy humans. The exact mechanism by which insulin release from the pancreas is stimulated by increased glucose levels is not fully understood, but the entry of glucose into the β-cells of the pancreas is required. Glucose enters the pancreatic β-cells by facilitated transport and is then phosphorylated by glucokinase. Expression of glucokinase is primarily limited to cells and tissues involved in the regulation of glucose metabolism, such as the liver and the pancreatic β-cells. The capacity of sugars to undergo phosphorylation and subsequent glycolysis correlates closely with their ability to stimulate insulin release. It is noted that not all tissues are dependent on insulin for glucose uptake. For example, the brain, kidneys and red blood cells are insulin independent tissues, while the liver, adipose and muscle are insulin dependent tissues.

When evoked by the presence of glucose (e.g., after a meal is ingested) in a non-diabetic individual, insulin secretion is biphasic: shortly after ingesting food, the pancreas releases the stored insulin in a burst, called a first phase insulin response, and then approximately 15-20 minutes later outputs further insulin to control the glycemic level from the food. The first phase insulin response reaches a peak after 1 to 2 minutes and is short-lived, whereas the second phase of secretion has a delayed onset but a longer duration. Thus, secretion of insulin rises rapidly in normal human subjects as the concentration of blood glucose rises above base levels (e.g., 100 mg/100ml of blood), and the turn-off of insulin secretion is also rapid, occurring within minutes after reduction in blood glucose concentrations back to the fasting level.

Diabetes Mellitus ("diabetes") is a disease state in which the pancreas does not release insulin at levels capable of controlling blood glucose and/or in which muscle, fat and liver cells respond poorly to normal insulin levels due to insulin resistance. Diabetes thus can result from a dual defect of insulin resistance and "burn out" of the β-cells of the pancreas. Diabetes Mellitus is classified into two types: Type 1 and Type 2. Approximately 5 to 10% of diagnosed diabetes cases are attributed to Type 1 diabetes, and approximately 90% to 95% are attributed to Type 2 diabetes.

Type 1 diabetes is insulin dependent and usually first appears in young people. In Type 1 diabetes, the islet cells of the pancreas stop producing insulin mainly due to autoimmune destruction, and the patient must self-inject the missing hormone. For type 1 diabetics, insulin therapy is essential to replace the absent endogenous insulin with an exogenous insulin supply.

Type 2 diabetes is a chronic and progressive disease that is commonly referred to as adult-onset diabetes or non-insulin dependent diabetes and may be caused by a combination of insulin resistance (or decreased insulin sensitivity) and, in later stages, insufficient insulin secretion. This is the most common type of diabetes in the Western world. Close to 6% of the adult population of various countries around the world, including the United States, have Type 2 diabetes, and about 30% of these patients will need exogenous insulin at some point during their lifespans due to secondary pancreatic exhaustion and the eventual cessation of insulin production. For type 2 diabetics, therapy has

consisted first of oral antidiabetic agents, which increase insulin sensitivity and/or insulin secretion, and only then insulin, if and when the oral agents fail.

Diabetes is the sixth leading cause of death in the United States, although this figure is likely an underestimate because complications resulting from diabetes are a major cause of morbidity in the population. Diabetes is associated with considerable morbidity and mortality in the form of cardiovascular disease, stroke, digestive diseases, infection, metabolic complications, ophthalmic disorders, neuropathy, kidney disease and failure, peripheral vascular disease, ulcerations and amputations, oral complications, and depression. Thus, diabetes contributes to many deaths that are ultimately ascribed to other causes.

The main cause of mortality with Diabetes is long term micro- and macro-vascular disease. Cardiovascular disease is responsible for up to 80% of the deaths of type 2 diabetic patients, and diabetics have a two- to four-fold increase in the risk of coronary artery disease, equal that of patients who have survived a stroke or myocardial infarction. In other words, heart disease, high blood pressure, heart attacks and strokes occur two to four times more frequently in adult diabetics than in adult non-diabetics. This increased risk of coronary artery disease combined with an increase in hypertensive cardiomyopathy manifests itself in an increase in the risk of congestive heart failure. These vascular complications lead to neuropathies, retinopathies and peripheral vascular disease. Diabetic retinopathy (lesions in the small blood vessels and capillaries supplying the retina of the eye, i.e., the breakdown of the lining at the back of the eye) is the leading cause of blindness in adults aged 20 through 74 years, and diabetic kidney disease, e.g., nephropathy (lesions in the small blood vessels and capillaries supplying the kidney, which may lead to kidney disease, and the inability of the kidney to properly filter body toxins), accounts for 40% of all new cases of end-stage renal disease (kidney failure). Diabetes also causes special problems during pregnancy, as the rate of congenital malformations can be five times higher in the children of women with diabetes, and diabetes is also the leading cause for amputation of limbs in the United States.

Poor glycemic control contributes to the high incidence of these complications, and the beneficial effects of tight glycemic control on the chronic complications of diabetes are widely accepted in clinical practice. However, only recently has it been firmly established that elevated blood glucose levels are a direct cause of long-term complications of diabetes. The Diabetes Control and Complications Trial (DCCT) and the United Kingdom Prospective Diabetes Study (UKPDS) both showed that control of blood glucose at levels as close to normal as possible prevents and retards development of diabetic retinopathy, nephropathy, neuropathy and microvascular disease.

Insulin resistance (or decreased insulin sensitivity) is also prevalent in the population, especially in overweight individuals, in those with risk of diabetes (i.e., pre-diabetic, wherein blood glucose levels are higher than normal but not yet high enough to be diagnosed as diabetes) and in individuals with type 2 diabetes who produce enough insulin but whose tissues have a diminished ability to adequately respond to the action of insulin. When the liver becomes insulin-resistant, the mechanism by which insulin affects the liver to suppress its glucose production breaks down, and the liver continues to produce glucose, even under hyperinsulinemic conditions (elevated plasma insulin levels). This lack of suppression can lead to a hyperglycemia (elevated blood glucose levels), even in a fasting state.

Insulin resistance plays an important role in the pathogenesis of hyperglycemia in type 2 diabetes, eventually inducing the development of diabetic complications. Furthermore, insulin resistance ostensibly plays a role in the pathogenesis of macrovascular disease, cardiovascular diseases and microvascular disease. Research currently shows that insulin resistance reaches a maximum and then plateaus, after which it is believed not to get appreciably worse but can improve.

In order to compensate and to overcome the insulin resistance, the pancreatic β-cells initially increase their insulin production such that insulin resistant individuals often have high plasma insulin levels. This insulin is released into the portal vein and presented to the liver constantly or almost constantly. It is believed that the liver's constant exposure to high levels of insulin plays a role in increased insulin resistance and impaired glucose tolerance. After a period of high demand placed on the pancreatic β-cells, the cells start to decompensate and exhaust, and insulin secretion, or insulin secretory capacity, is reduced at later stages of diabetes. It is estimated that, by the time an individual is diagnosed with type 2 diabetes, roughly 50% of the β-cells have already died due to increased demand for insulin production.

Present treatment of insulin resistance and impaired glucose tolerance (IGT) involves sensible lifestyle changes, including weight loss to attain healthy body weight, 30 minutes of accumulated moderate-intensity physical activity per day and diet control, including increased dietary fiber intake and regulation of blood sugar levels and of caloric intake. Intensive lifestyle modifications and medication interventions can delay or prevent a patient's progression from a state of impaired glucose tolerance to type 2 diabetes. The Diabetes Prevention Program has demonstrated that, compared with placebo intervention, intensive lifestyle intervention reduced the incidence of type 2 diabetes by 58%, and medication intervention (specifically using metformin, discussed below) reduced the incidence of type 2 diabetes by 31 % over 2.8 years.

Oral administrable drugs currently available for improving a patient's insulin resistance and for management of type 2 diabetes fall into two general categories: those that increase insulin supply (sulfonylureas, other secretagogues

and insulin itself) and those that decrease insulin resistance or improve its effectiveness (biguanides, thiazolidinediones).

Oral sulfonylurea secretagogues are believed to interact with ATP-sensitive potassium channels in the β-cell membrane to stimulate the pancreas to increase secretion of insulin, and D-phenylalanine derivatives help the pancreas make more insulin quickly. Typically, such secretagogues are useful for increasing insulin levels sufficiently to achieve desired basal insulin levels in patients with early stages of type II diabetes, who are still able to produce their own insulin, but not likely for increasing insulin levels sufficiently to achieve desired basal insulin levels in patients with later stages of type II diabetes, who have very little pancreatic function left and produce very little insulin endogenously. In such patients, the basal insulin levels are achieved, e.g., via the use of subcutaneous injections of insulin.

Oral hypoglycemic agents that improve a patient's insulin resistance, such as thiazolidinediones, which make the patient more sensitive to insulin, and biguanides, which decrease the amount of glucose made by the liver, are currently available clinically for patients with type 2 diabetes and insulin resistance. Thiazolidinediones improve sensitivity to insulin in muscle and adipose tissue and inhibit hepatic gluconeogenesis, and thus depend on the presence of insulin for their action. The two currently approved thiazolidinedione compounds are pioglitazone (Actos® by Takeda Pharmaceuticals America, Inc. of Lincolnshire, IL) and rosiglitazone (Avandia® by GlaxoSmithKline of Research Triangle Park, NC). Biguanides, such as Metformin (Glucophage® and Glucophage® XR by Bristol-Myers Squibb Company of Princeton, NJ), which is the only biguanide available for therapeutic use, decreases hepatic glucose production (gluconeogenesis), decreases intestinal absorption of glucose and improves insulin sensitivity by increasing peripheral glucose uptake and utilization.

Oral antidiabetic monotherapy, while initially successful in reducing hyperglycemia, seldom succeeds for more than a few years. In many patients with type 2 diabetes, oral antidiabetic monotherapy does not sufficiently control glycemia in the long-term, leading to a requirement for multiple therapies. Even with combinations of antidiabetic oral agents, many diabetic patients eventually require insulin treatment in order to administer enough insulin such that the patient will have normal carbohydrate metabolism throughout the day.

Because the pancreas of a diabetic individual does not secrete sufficient insulin throughout the day, in order to effectively control diabetes through insulin therapy, a long-lasting insulin treatment, known as basal insulin, must be administered to provide the slow and steady release of insulin that is needed to control blood glucose concentrations and to keep cells supplied with energy when no food is being digested. In addition, a bolus, fast-acting treatment must also be administered at those times of the day when the patient's blood glucose level tends to rise too high, such as at meal times. Alternative to administering basal insulin in combination with bolus insulin, repeated and regular lower doses of bolus insulin may be administered in place of the long-acting basal insulin, and bolus insulin may be administered postprandially as needed.

Because insulin is a peptide drug that is not absorbed intact in the gastrointestinal tract, it ordinarily requires parenteral administration such as by subcutaneous injection. Thus, most diabetic patients self-administer insulin by subcutaneous injections, often multiple times per day. However, the limitations of multiple daily injections, such as pain, inconvenience, frequent blood glucose monitoring, poor patient acceptability, compliance and the difficulty of matching postprandial insulin availability to postprandial glucose-control requirements, are some of the shortcomings of insulin therapy. In addition, there is also the potential for hypoglycemia if the administered insulin provides a therapeutic effect over too great a time, e.g., after the rise in glucose levels that occur as a result of ingestion of the meal has already been lowered. These have resulted in the generally inadequate glycemic control believed to be associated with many of the chronic complications (comorbidities) associated with diabetes.

In addition, hyperinsulinemia (elevated blood concentrations of insulin) can also occur, such as by the administration of insulin in a location (and manner) that is not consistent with the normal physiological route of delivery. Insulin circulates in blood as the free monomer, and its volume of distribution approximates the volume of extracellular fluid. Under fasting conditions, the concentration of insulin in portal blood is, e.g., about 2-4 ng/mL, whereas the systemic (peripheral) concentration of insulin is, e.g., about 0.5 ng/mL, in normal healthy humans, translating into, e.g., a 5:1 ratio. In human diabetics who receive insulin via subcutaneous injection, the portal vein to periphery ratio is changed to about 0.75:1. Thus, in such diabetic patients, the liver does not receive the necessary concentrations of insulin to adequately control blood glucose, while the peripheral circulation is subjected to higher concentrations of insulin than are found in healthy subjects. Elevated systemic levels of insulin may lead to increased glucose uptake, glycogen synthesis, glycolysis, fatty acid synthesis, cortisol synthesis and triacylglycerol synthesis, leading to the expression of key genes that result in greater utilization of glucose.

Thus, it has long been desirable to create compositions of insulin that do not alter physiological clinical activity and that do not require injections. Oral delivery of insulin is a particularly desirable route of administration, for safety and convenience considerations, because it can minimize or eliminate the discomfort that often attends repeated hypodermic injections.

Oral delivery of insulin may also have advantages beyond convenience, acceptance and compliance issues. Insulin absorbed in the gastrointestinal tract is thought to mimic the physiologic route of insulin secreted by the pancreas because both are released into the portal vein and carried directly to the liver before being delivered into the peripheral

circulation. Absorption into the portal vein maintains a peripheral-portal insulin gradient that regulates insulin secretion. In its first passage through the liver, roughly 60% of the insulin is retained and metabolized, thereby reducing the incidence of peripheral hyperinsulinemia, a factor linked to complications in diabetes. However, insulin absorption in the gastrointestinal tract is prevented presumably by its molecular size and its susceptibility for enzymatic degradation. The physicochemical properties of insulin and its susceptibility to enzymatic digestion have precluded the design of a commercially viable oral or alternate delivery system.

Emisphere Technologies, Inc. has developed compositions of insulin that are orally administrable, e.g., absorbed from the gastrointestinal tract in adequate concentrations, such that the insulin is bioavailable and bioactive following oral administration and provide sufficient absorption and pharmacokinetic/pharmacodynamic properties to provide the desired therapeutic effect, i.e., cause a reduction of blood glucose, as disclosed in U.S. Patent Applications Nos. 10/237,138, 60/346,746, 60/347,312, 60/368,617, 60/374,979, 60/389,364, 60/438,195, 60/438,451, 60/578,967, 60/452,660, 60/488,465, 60/518,168, 60/535,091 and 60/540,462, as well as in International Patent Application Publications Nos. WO 03/057170, WO 03/057650 and WO 02/02509 and International Patent Application No. PCT/US04/00273, all assigned to Emisphere Technologies, Inc., all of which are incorporated herein by reference.

The novel drug delivery technology of Emisphere Technologies, Inc. is based upon the design and synthesis of low molecular weight compounds called "delivery agents" that, when formulated with insulin, increase the oral bioavailability of insulin by facilitating the transport of insulin across the gastrointestinal wall to enable its gastrointestinal absorption. It is believed that the delivery agent, which is not intended to possess any inherent pharmacological activity, interacts with insulin non-covalently, creating more favorable physical-chemical properties for absorption. Once across the gastrointestinal wall, insulin disassociates rapidly from the delivery agent and reverts to its normal, pharmacologically active state. In clinical human studies, the delivery agent was shown to enhance the gastrointestinal absorption of insulin following oral administration so as to reduce blood glucose concentrations in both healthy subjects and diabetic patients.

It is well documented that type 2 diabetes results from two impairments: a relative insulin deficiency accompanied by insulin resistance. Oral antidiabetic monotherapies directly address only one defect as their primary mechanism of action, and do not control blood glucose sufficiently well to meet current glycemic targets. Generally, there is accruing evidence that combination therapies, which affect both defects of diabetes, insulin deficiency and insulin resistance, are advantageous and superior to monotherapy. However, the free co-administration of two or more oral antidiabetic drugs tends to complicate therapeutic regimens, with the associated risk of impaired compliance with therapy.

Single-dosage combination therapies provide an alternative therapeutic option for the delivery of multiple therapies combination therapy for the management of type 2 diabetes without adding to the burden of polypharmacy that is commonly faced by these patients. Careful selection of the components of a combination tablet is essential, as the agents to be combined must have complementary mechanisms of action that address the underlying pathophysiology of the disease and must have complementary pharmacokinetic properties that support their co-administration. Single-dose combination therapies provide a means of intensifying antidiabetic therapy while supporting good patient compliance and at present represent a largely unexplored opportunity to improve the management of type 2 diabetes. For example, the Food and Drug Administration has only recently approved a new combination glyburide/metformin hydrochloride tablet (Glucovance®, by Bristol-Myers Squibb Company, Princeton, NJ) that comprises a sulfonyurea (insulin secretagogue) and the biguanide metformin (an insulin sensitizer) for just such a rationale.

Thus, simultaneous initiation of complementary compounds that address the two known impairments of type 2 diabetes is logical. By treating both impairments early, better glycemic control may be achieved, ultimately resulting in a reduction in chronic complications. Insulin, the naturally occurring endogenous hormone, is the most effective and has the most durable effects in achieving and maintaining normoglycemia. Insulin, however, addresses only the single defect of insulin deficiency and was until recently unavailable by oral administration. Combining insulin with a drug that reduces insulin resistance can be a more efficient treatment to approach and/or reach normoglycemia. Most studies show that better glycemic control is achieved with insulin combination therapy than with insulin alone in previously insulin-treated patients. Combination regimens also allow use of fewer insulin injections, which may ease titration of the insulin dose and increase compliance (See Yki-Jarvinen H et al, Ann Intern Med 130:389-396 (1999); Yki-Jarvinen H et al., N Engl J Med 327:1426-1433 (1992); and Lindstrom T, et al., Diabet Med 16:820-826 (1999).

Among the oral hypoglycemic agents, a biguanide, specifically metformin, is suitable for combination therapy with insulin. Significantly, with regard to weight gain and its associated morbidities and in insulin resistance, which is a major concern diabetics who begin antidiabetic pharmacotherapy, metformin is the only antidiabetic agent that has consistently shown not to result in weight gain in treated patient. Better still, metformin treatment is often associated with reductions in body weight in overweight patients, and with improvements in lipid profiles in dyslipidemic patients. In the UKPDS, patients in intensive therapy gained more weight than those in conventional therapy groups, as patients taking insulin gained an average of 4 kg, compared to an average 2.6 kg weight gain for those on chlorpropamide (Diabinese®) and an average 1.7 kg weight gain for those on glibenclamide (glyburide [Micronase®]). See, UKPDS, Lancet 353:837-853 (1998). Yet patients in the intensive therapy groups also had fewer microvascular complications, suggesting that tight glycemic control may be more important in therapeutic decision-making. The use of metformin

(Glueophage®) as an adjunct to insulin therapy provides effective glycemic control without significant weight gain. See Yki-Jarvinen H, Ann Intern Med 131:182-188 (1999); and Avilés-Santa L, Ann Intern Med 131:182-188 (1999).

Besides its advantage in the area of weight control, metformin is to date still the only oral antidiabetic agent proven to reduce the total burden of microvascular and macrovascular diabetic complications and to prolong the lives of type 2 diabetic patients. The UKPDS evaluated the effects of metformin on glycemic control and clinical outcomes in overweight type 2 diabetic patients with hyperglycemia despite previous treatment with diet and exercise, over a median follow-up period of 10 years. Compared with conventional, diet-based treatment, intensive glycemic management with metformin was associated with significant reductions in the incidence of diabetes-related and all-cause mortality ($p = 0.017$ and $p = 0.011$, respectively), any diabetes-related endpoint ($p = 0.002$) and myocardial infarction ($p = 0.01$).

It is, therefore, desirable to provide oral pharmaceutical compositions of insulin and an antidiabetic agent such as a biguanide, preferably metformin, to provide a protocol for insulin treatment for patients with impaired glucose tolerance or with early stage or late stage diabetes, which treatment can be administered orally multiple times daily, such as at or shortly prior to mealtime and/or at or shortly prior to bedtime, and has positive and long lasting effects on the patient's glucose tolerance, glycemic control, insulin secretory capacity and insulin sensitivity, but does not increase the risk of hypoglycemia, hyperinsulinemia and weight gain that are normally associated with insulin therapy treatments.

**SUMMARY OF THE INVENTION**

It is an object of the present invention to provide an oral dosage form comprising a pharmaceutical composition comprising insulin and an antidiabetic agent such as a biguanide, preferably metformin.

It is another object of the present invention to provide a therapeutic oral treatment of insulin and an antidiabetic agent such as a biguanide, preferably metformin, for patients with impaired glucose tolerance or with early stage or late stage diabetes to provide positive and long lasting therapeutic effects on the patient's glucose tolerance and glycemic control and therapeutic effects to the patient greater than or unseen in current parenteral insulin therapy.

It is another object of the present invention to provide a therapeutic oral treatment of insulin and an antidiabetic agent such as a biguanide, preferably metformin, for patients with impaired glucose tolerance or with early stage or late stage diabetes to improve the patient's endogenous capacity to handle sugar load and to provide for the patient a decreased fasting blood glucose concentration when compared with the patient's own baseline level prior to starting the treatment.

It is yet another object of the present invention to provide a therapeutic oral treatment of insulin and an antidiabetic agent such as a biguanide, preferably metformin, for patients with impaired glucose tolerance or with early stage or late stage diabetes to improve the insulin utilization, insulin sensitivity and insulin secretion capacity of the patient's body.

It is a further object of the present invention to provide a therapeutic oral treatment of insulin and an antidiabetic agent such as a biguanide, preferably metformin, for patients with impaired glucose tolerance or with early stage or late stage diabetes without the negative side effects currently seen in parenteral insulin therapy, without inducing hypoglycemia or hyperinsulinemia, without the weight gain commonly associated with parenteral insulin therapy, and with a reduction in the need for frequent monitoring of blood sugar levels currently needed with current insulin therapy regimens.

It is still another object of the present invention to provide a therapeutic oral treatment of insulin and an antidiabetic agent such as a biguanide, preferably metformin, for patients who are failing dual or multiple therapy with sulfonureas and insulin sensitizers.

It is yet another object of the present invention to provide pharmaceutical compositions for oral administration comprising insulin, a delivery agent that facilitates insulin transport in a therapeutically effective amount to the bloodstream and an antidiabetic agent, such as a biguanide, preferably metformin, which compositions are therapeutically and quickly effective.

It is a further object of the present invention to provide therapeutically effective pharmaceutical compositions for oral administration of insulin, a delivery agent that facilitates insulin transport in a therapeutically effective amount to the bloodstream and an antidiabetic agent such as a biguanide, preferably metformin, for the treatment of diabetes, for the treatment of impaired glucose tolerance, for the purpose of achieving glucose homeostasis, for the treatment of early stage diabetes, for the treatment of late stage diabetes, and/or to serve as replacement therapy for type I diabetic patients to provide longer lasting effects on the patient's glucose tolerance and glycemic control without the risks of hypoglycemia, hyperinsulinemia and weight gain that are normally associated with insulin therapy treatments.

It is still a further object of the present invention to provide methods of treating mammals with impaired glucose tolerance, early stage diabetes or late stage diabetes, for achieving glucose homeostasis in mammals, for prophylactically sparing pancreatic β-cell function, for preventing β-cell death or dysfunction, for long term protection of a mammal from developing overt or insulin dependent diabetes, for delaying the onset of overt or insulin dependent diabetes in a mammal that has impaired glucose tolerance or early stage diabetes, and for reducing the incidence and/or severity of systemic hyperinsulinemia associated with chronic dosing of insulin or of one or more disease states associated with chronic dosing of insulin.

The invention also provides a method for treating a mammal with impaired glucose tolerance or with early or late stage diabetes, comprising orally administering to a mammal a therapeutically effective dose of a pharmaceutical formulation comprising insulin, a delivery agent and an antidiabetic agent such as a biguanide, preferably metformin, such that the mammal achieves improved glucose tolerance and glycemic control as compared with baseline levels prior to treatment, without any statistically significant weight gain by the mammal over the treatment period, and without any statistically significant risk of hypoglycemia or hyperinsulinemia in the mammal over the treatment period.

The present invention provides methods of treating mammals with impaired glucose tolerance, early stage diabetes and late stage diabetes; for achieving glucose homeostasis; for reducing the incidence and/or severity of systemic hyperinsulinemia associated with chronic dosing of insulin. It is believed that the present invention also provides methods for reducing the incidence and/or severity of one or more disease states associated with chronic dosing of insulin; for prophylactically sparing β-cell function or for preventing β-cell death or dysfunction, in a mammal which has impaired glucose tolerance or early stage diabetes mellitus; and for long-term protection from developing overt or insulin dependent diabetes, or for delaying the onset of overt or insulin dependent diabetes, in a mammal which has impaired glucose tolerance or early stage diabetes.

In a preferred embodiment of the invention, such methods comprise orally administering a therapeutically effective dose of a pharmaceutical formulation comprising insulin, a delivery agent that facilitates the absorption of the insulin from the gastrointestinal tract and a biguanide such as metformin, to provide a therapeutically effective reduction in blood glucose and a plasma insulin concentration, to provide a therapeutically effective reduction and/or control in blood glucose concentration and a plasma insulin concentration that is reduced relative to the plasma insulin concentration provided by a therapeutically equivalent dose of subcutaneously injected insulin. The determination of insulin concentration obtained in patients who have been administered subcutaneous insulin are well known to those skilled in the art.

Preferably, the dosage form of the present invention will be administered for at least one day, more preferably on a chronic basis, and can be administered for the life of the patient. Most preferably, the dosage form of the present invention will be administered on a chronic basis, e.g., for at least about two weeks.

In a preferred embodiment, administration of the pharmaceutical formulation takes place multiple times daily, preferably at bedtime and preprandially during the day time, e.g., preprandially for breakfast, lunch and dinner. More preferably, administration of the pharmaceutical formulation is at or shortly prior to bedtime and concurrently with or shortly prior to ingestion of a meal, i.e., within about 15 minutes or less of ingestion of the meal.

In another preferred embodiment of the invention, the oral pharmaceutical formulation will be administered about once daily to about four times daily, preprandially and/or at bedtime, depending upon the extent of the patient's impaired glucose tolerance and need for exogenous glycemic control. If the patient has a need for fasting glycemic control, the oral pharmaceutical formulation will be administered only at or shortly prior to bedtime. If the patient has a need for post-prandial glycemic control, the oral pharmaceutical formulation will be administered preprandially for all meals. If the patient has a need for comprehensive glycemic control, the oral pharmaceutical formulation will be administered preprandially for all meals and at or shortly prior to bedtime.

Preferably, the therapeutic insulin treatment of the present invention will be administered to patients having some form of impaired glucose tolerance, e.g., to patients with an $HbA_1c$ ranging from normal to elevated levels. This can range from insulin resistance seen in pre-diabetics and early stage Type 2 diabetics to failure of insulin production by the pancreas seen in Type 1 diabetes and late stage Type 2 Diabetes. More particularly, the treatment can be administered to anyone in the range of normal glycemic control to impaired glycemic control to late stage type 2 diabetes or type 1 diabetes.

In certain preferred embodiments, the mammal achieves improved glucose tolerance after the treatment, as demonstrated by one of the following:

- better endogenous capacity of the mammal to handle sugar load as measured by blood glucose concentration, following a sugar load, that is reduced by a statistically significant amount as compared with baseline blood glucose concentration, following a glucose load, prior to treatment.
- better endogenous capacity of the mammal to handle sugar load as measured by an AUC of blood glucose excursion, following a glucose load, that is reduced by a statistically significant amount as compared with AUC of blood glucose excursion, following a glucose load, prior to treatment.
- decreased fasting blood glucose levels as measured by fasting blood glucose concentration that is reduced by a statistically significant amount as compared with baseline fasting blood glucose concentration prior to treatment.
- decreased serum fructosamine levels, as measured by serum fructosamine assay, that is reduced by a statistically significant amount as compared with baseline serum fructosamine levels prior to treatment.
- decreased HbA1c levels, as measured by a statistically significant decline in HbA1c levels after treatment compared with baseline levels prior to treatment.

In certain embodiments, the mammal achieves improved glycemic control, as measured by a reduced serum

fructosamine concentration compared with baseline levels prior to treatment.

In certain preferred embodiments, the mammal achieves improved insulin utilization, insulin sensitivity and insulin secretion capacity after the treatment as compared with baseline levels prior to treatment. Preferably, the improved insulin utilization and insulin sensitivity are measured by a statistically significant decline in HOMA (Homeostasis Model Assessment), and the improved insulin secretion capacity is measured by a statistically significant decline in Stumvoll first-phase insulin secretion capacity index.

In certain preferred embodiments, the mammal achieves improved glucose tolerance, glycemic control, insulin utilization, insulin sensitivity and insulin secretion capacity as compared with baseline levels prior to treatment, without any statistically significant weight gain by the mammal over the treatment period, and without any statistically significant risk of hypoglycemia or hyperinsulinemia in the mammal over the treatment period.

In preferred embodiments of the oral dosage forms of the invention described above, the oral dosage form is solid, and is preferably provided incorporated within a gelatin capsule or contained in a tablet.

In certain preferred embodiments, the dose of unmodified insulin contained in one or more dosage forms is from about 50 Units to about 600 Units (from about 2 to about 23 mg), preferably from about 100 Units (3.8 mg) to about 450 Units (15.3 mg) insulin, more preferably from about 200 Units (7.66 mg) to about 350 Units (13.4 mg), and still more preferably about 300 Units (11.5 mg), based on the accepted conversion of factor of 26.11 Units per mg.

In certain preferred embodiments, the dosage forms of the invention provide a $t_{max}$ for insulin at from about 5 minutes to about 30 minutes, and more preferably at from about 10 minutes to about 25 minutes after oral administration to diabetic patients. In certain preferred embodiments of the invention, the dosage forms begin delivering insulin into the systemic circulation to produce a peak plasma insulin concentration at about 10 to about 20 minutes post oral administration and in further preferred embodiments, a peak plasma insulin concentration at about 10 minutes to about 15 minutes post oral administration to patients who ingested the dosage at about 0 or about 10 minutes prior to ingestion of a meal.

Because insulin entry into the bloodstream produces a decrease in blood glucose levels, oral absorption of insulin may be verified by observing the effect on a subject's blood glucose following oral administration of the composition. The magnitude of the decrease in blood glucose produced by insulin absorbed into the bloodstream following entry into the gastrointestinal tract varies with the dose of insulin. In certain preferred embodiments, the composition provides a $t_{max}$ for maximum control of glucose excursion at about 0.25 to about 1.5 hours, more preferably at about 0.75 to about 1.25 hours, after oral administration. In certain preferred embodiments, the $t_{max}$ for post-prandial glucose control occurs preferably at less than about 120 minutes, more preferably at less than about 80 minutes, and still more preferably at about 45 minutes to about 60 minutes, after oral administration of the composition.

In certain preferred embodiments, the pharmaceutical composition contained in one or more dosage forms comprises from about 5 mg to about 800 mg of delivery agent, preferably about 20 mg to about 600 mg, more preferably from about 30 mg to about 400 mg, even more preferably from about 40 mg to about 200 mg, most preferably about 40 mg, 80 mg or 160 mg.

For purposes of the present invention, a preferred delivery agent is identified via chemical nomenclature as 4-[(4-chloro, 2-hydroxybenzoyl)amino]butanoic acid. In certain preferred embodiments, the delivery agent is a sodium salt, preferably monosodium salt. Alternatively, the same compound is identified by the alternative nomenclature monosodium N-(4-chlorosalicyloyl)-4-aminobutyrate, or by the short name "4-CNAB".

For purposes of the present invention, a preferred antidiabetic agent to be co-administered with insulin and the delivery agent is a hypoglycemic agent, specifically a biguanide. In preferred embodiments of the invention, the biguanide is metformin or, more particularly, metformin, hydrochloride. It is anticipated that combining insulin and a delivery agent with the biguanide metformin will enhance the therapeutic benefit of oral insulin, will facilitate attainment of normoglycemia, will result in reduction in $HbA_1c$ levels within the desired goal, reduce insulin resistance, spare pancreatic β-cells, delay or prevent the progression of diabetes, and defer or eliminate the need to resort to injectable insulin. An oral combination comprising insulin, a delivery agent and metformin is likely to offer great advantages in the management of patients with type 2 diabetes.

The following terms will be used throughout the application as defined below:

Patient -- refers to any mammal in whom there is determined to be.

Diabetic patient -- refers to a mammal with a form of pre-diabetes or diabetes, either diagnosed or undiagnosed, and/or with a condition that would respond to an anti-diabetic and/or insulin treatment.

Mammal -- includes but is not limited to rodents, aquatic mammals, domestic animals such as dogs and cats, farm animals such as sheep, pigs, cows and horses, and preferably humans.

Diabetes or Diabetes Mellitus -- is deemed to encompass type 1 and type 2 diabetes mellitus, unless specifically specified otherwise.

Overt Diabetes -- is deemed to encompass type 1 and type 2 diabetes mellitus that is insulin dependent.

Early stage diabetes -- refers to a condition of impaired glycemic control, absent treatment, wherein the function of the islet cells of the pancreas still exist, although in an impaired state, also including impaired glucose tolerance (IGT)

and impaired fasting blood glucose (IFG), e.g., the patient's endogenous insulin production is insufficient to provide a first phase insulin response following ingestion of a meal but is sufficient to provide a second phase insulin response following ingestion of a meal.

Late stage diabetes -- refers to a condition of impaired glycemic control, absent treatment, wherein the islet cells of the pancreas are approaching or have reached total failure, e.g., the patient's endogenous insulin production is insufficient to provide a first or a second phase insulin response following ingestion of a meal.

Treatment -- when used herein with respect to diabetes is deemed to include prevention of diabetes, delay of the onset of diabetes, delay of worsening of diabetic conditions and delay of progression from an earlier stage of diabetes to a later stage of diabetes, unless specifically specified otherwise.

Delivery agent -- refers to carrier compounds or carrier molecules that are effective in the oral delivery of therapeutic agents, and may be used interchangeably with "carrier".

Active or Active agent -- refers to the active agents disclosed or incorporated by reference herein, including, for example, but not limited to, insulin and metformin,

Effective amount of active agent -- refers to the amount of the active agent, its salt or salts, including its solvates, active metabolites, prodrugs, or racemates or enantiomers thereof, that, when administered to a mammal for treating or preventing a state, disorder or condition is sufficient to effect such treatment or prevention, and will vary depending on the active ingredient, the state, disorder, or condition to be treated and its severity, and the age, weight, physical condition and responsiveness of the mammal to be treated.

Therapeutically effective amount of insulin -- refers to an amount of insulin included in the dosage forms of the invention which is sufficient to achieve a clinically relevant control of blood glucose concentrations in a human diabetic patient either in the fasting state or in the fed state effective, during the dosing interval.

Effective amount of delivery agent -- refers to an amount of the delivery agent that has been shown to deliver the therapeutic agent or to promote the absorption of a desired amount of the therapeutic agent from, for example, the gastrointestinal tract, following oral administration by measurement of pharmacokinetic and/or pharmacodynamic endpoints.

An "effective amount of the pharmaceutical formulation" is an amount of the pharmaceutical formulation described which is effective to treat or prevent a condition in a subject to whom it is administered over some period of time, e.g., provides a therapeutic effect during a desired dosing interval. Generally, an effective amount of the pharmaceutical formulation includes amounts of insulin and metformin and at least one delivery agent to treat or prevent the desired condition over a desired period of time (i.e., an effective amount of delivery agent and an effective amount of insulin and metformin).

Organic solvents -- refers to any solvent of non-aqueous origin, including liquid polymers and mixtures thereof. Organic solvents suitable for the present invention include: acetone, methyl alcohol, methyl isobutyl ketone, chloroform, 1-propanol, isopropanol, 2-propanol, acetonitrile, 1-butanol, 2-butanol, ethyl alcohol, cyclohexane, dioxane, ethyl acetate, dimethylformamide, dichloroethane, hexane, isooctane, methylene chloride, tert-butyl alchohol, toluene, carbon tetrachloride, or combinations thereof.

Peptide -- refers to a polypeptide of small to intermediate molecular weight, usually 2 or more amino acid residues and frequently but not necessarily representing a fragment of a larger protein.

Protein -- refers to a complex high polymer containing carbon, hydrogen, oxygen, nitrogen and usually sulfur and composed of chains of amino acids connected by peptide linkages. Proteins in this application refer to glycoproteins, antibodies, non-enzyme proteins, enzymes, hormones and sub-units of proteins, such as peptides. The molecular weight range for proteins includes peptides of 1000 Daltons to glycoproteins of 600 to 1000 kiloDaltons.

Reconstitution -- refers to dissolution of compositions or compositions in an appropriate buffer or pharmaceutical composition.

Dosage Form -- refers to a physically discrete unit suitable for human and animal subjects and packaged individually as is known in the art. It is contemplated for purposes of the present invention that dosage forms of the present invention comprising chosen therapeutically effective amounts of active agent or agents may include one or more unit doses (e.g., tablets, capsules, powders, semisolids (e.g. gelcaps or films), liquids for oral administration, ampoules or vials for injection, loaded syringes) to achieve the therapeutic effect. It is further contemplated for the purposes of the present invention that a preferred embodiment of the dosage form is an oral dosage form.

The term "multiple dose" means that the patient has received at least two doses of the drug composition in accordance with the dosing interval for that composition.

The term "single dose" means that the patient has received a single dose of the drug composition or that the repeated single doses have been administered with washout periods in between.

Unless specifically designated as "single dose" or at "steady-state" the pharmacokinetic parameters disclosed and claimed herein encompass both single dose and multiple-dose conditions.

Unmodified insulin -- refers to insulin prepared in any pharmaceutically acceptable manner or from any pharmaceutically acceptable source which is not conjugated with an oligomer such as that described in U.S. Patent No.

6,309,633 and/or which not has been subjected to amphiphilic modification such as that described in U.S. Patent Nos. 5,359,030; 5,438,040; and/or 5,681,811, which patents are hereby incorporated by reference in their entireties.

The phrase "equivalent therapeutically effective reduction" as used herein means that a maximal reduction of blood glucose concentration achieved by a first method of insulin administration (e.g. via oral administration of insulin in a patient(s)) is not more than 20%, and preferably not more than 10% and even more preferably not more than 5% different from a maximal reduction of blood glucose concentration after administration by a second method (e.g., subcutaneous injection) in the same patient(s) or a different patient requiring the same reduction in blood glucose level. The phrase may also mean the dose required to approximate normoglycemia by any method of administration, normoglycemia being defined as variability from a subject's baseline blood glucose of not more than 20%, preferably 10%, more preferably 5%, in the fasted state.

The term "meal" as used herein means a standard, ADA and/or a mixed meal.

The term "mean", when preceding a pharmacokinetic value (e.g., mean $t_{max}$), represents the arithmetic mean value of the pharmacokinetic value unless otherwise specified.

The term "mean baseline level" as used herein means the measurement, calculation or level of a certain value that is used as a basis for comparison, which is the mean value over a statistically significant number of subjects, e.g., across a single clinical study or a combination of more than one clinical study.

The term "$C_{max}$" as used herein is the highest plasma concentration of the drug or delivery agent observed within the sampling interval.

The term "$t_{max}$" as used herein is the time post-dose at which $C_{max}$ is observed.

The term "AUC" as used herein means area under the plasma concentration-time curve, as calculated by the trapezoidal rule over the complete sample collection interval.

The term "Bioavailability" as used herein means the degree or ratio (%) to which a drug or agent is absorbed or otherwise available to the treatment site in the body relative to a parenteral route. This is calculated by the formula

$$\text{Relative Bioavailability (\%)} = \frac{\text{Dose SC}}{\text{Dose Oral}} \text{ X } \frac{\text{AUC Oral}}{\text{AUC SC}} \text{ X } 100$$

The term "Biopotency" as used herein means the degree or ratio (%) to which a drug or agent is effective relative to a parenteral route. This is calculated by the formula

$$\text{Relative Biopotency (\%)} = \frac{\text{Dose SC}}{\text{Dose Oral}} \text{ X } \frac{\text{AUC Oral}}{\text{AUC SC}} \text{ X } 100$$

The term "nighttime" or "bedtime" as used herein means a time before the patient goes to sleep and is not limited to clock time or cycles of light and dark, and alternately refers to a time during a day or night of longest fast, a period without external glucose source.

For the purposes of the present specification, as used herein, the phrase administered "at nighttime" or "at or shortly before (prior to) bedtime" means administered less than about 3 hours, preferably less than about 2 hours and more preferably less than about 1 hour prior to a prolonged period of sleep, or relative physical and/or mental inactivity, and fast, e.g., overnight. Whereas overnight typically means from the late night (p.m.) hours to the early morning (a.m.) hours, it could mean any period of a sleep-wake cycle during which a person obtains his/her necessary period of sleep. For the purposes of the present specification, administration should also occur at least about one hour, preferably at least about 1.5 hours, more preferably at least about 2 hours and still more preferably at least about 2 to about 3 hours after the last meal of the day.

For the purposes of the present specification, as used herein, the phrase administered "at mealtime" or "at or shortly before (prior to) ingestion of a meal" means administered within about 30 minutes prior to the meal. For the purposes of the present specification, the administration is preferably within about 25 minutes, more preferably within about 20 minutes, even more preferably within about 15 minutes, still more preferably within about 10 minutes, further more preferably within about 5 minutes of ingestion of the meal, and most preferably administered concurrently with ingestion of the meal (within about 0 minutes).

As used herein and in the appended claims, the singular forms "a," "an," and "the," include plural referents

unless the context clearly indicates otherwise. Thus, for example, reference to "a molecule" includes one or more of such molecules, "a reagent" includes one or more of such different reagents, reference to "an antibody" includes one or more of such different antibodies, and reference to "the method" includes reference to equivalent steps and methods known to those of ordinary skill in the art that could be modified or substituted for the methods described herein.

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviations, per practice in the art. Alternatively, "about" with respect to the formulations can mean a range of up to 10%, preferably up to 5%.

The terms "alkyl", "alkenyl", "alkoxy", "alkylene", "alkenylene", "alkyl(arylene)", and "aryl(alkylene)" include, but are not limited to, linear and branched alkyl, alkenyl, alkoxy, alkylene, alkenylene, alkyl(arylene), and aryl(alkylene) groups, respectively.

The phrase "pharmaceutically acceptable" refers to compounds or compositions that are physiologically tolerable and do not typically produce an allergic or similar untoward reaction, such as gastric upset, dizziness and the like, when administered to a mammal.

As used herein, the term "treat" includes one or more of the following:

(a) arresting, delaying the onset (i.e., the clinical manifestation of a disorder) and/or reducing the risk of developing or worsening a disorder;
(b) relieving or alleviating at least one symptom of a disorder in a mammal, including for example, hyperglycemia;
(c) relieving or alleviating the intensity and/or duration of a manifestation of a disorder experienced by a mammal including ,but not limited to, those which are in response to a given stimulus (e.g., pressure, tissue injury or cold temperature); or
(d) prophylactically preventing, curing, healing, alleviating, relieving, altering, remedying, ameliorating, improving, or affecting a condition (e.g., a disease), the symptoms of the condition, or the predisposition toward the condition.

The term "sustained release" as used herein refers to the release of an active ingredient over an extended period of time leading to lower peak plasma concentrations and a prolonged $t_{max}$ as compared to "immediate release" formulations of the same active ingredient.

The term "polymorph" refers to a crystallographically distinct form of a substance.

The term "hydrate" as used herein includes, but is not limited to, (i) a substance containing water combined in the molecular form and (ii) a crystalline substance containing one or more molecules of water of crystallization or a crystalline material containing free water.

The term "solvate" as used herein includes, but is not limited to, a molecular or ionic complex of molecules or ions of a solvent with molecules or ions of a delivery agent or insulin and/or metformin salt.

Unless defined otherwise, all technical and scientific terms used herein have the same meaning as commonly understood by one of ordinary skill in the art to which the invention belongs. Although any methods, compositions, reagents, cells, similar or equivalent to those described herein can be used in the practice or testing of the invention, the preferred methods and materials are described herein. All publications mentioned herein are incorporated herein, including all figures, graphs, equations, illustrations, and drawings, to describe and disclose specific information for which the reference was cited in connection with.

The publications discussed above are provided solely for their disclosure before the filing date of the present application. Nothing herein is to be construed as an admission that the invention is not entitled to antedate such disclosure by virtue of prior invention. Throughout this description, the preferred embodiment and examples shown should be considered as exemplars, rather than as limitations on the present invention.

## BRIEF DESCRIPTION OF THE DRAWINGS

Figure 1 shows a plot of the whole blood glucose change in values from baseline for the oral delivery of insulin/ metformin in rodents;

Figure 2 shows a plot of percent change of blood glucose values from baseline for the oral delivery of insulin/ metformin in rodents; and

Figure 3 shows a plot of the for percent change from control of the oral delivery of insulin/metformin in rodents.

## DETAILED DESCRIPTION OF THE INVENTION

By virtue of the chronic administration of oral dosage forms of the present invention instead of equi-effective subcutaneous doses of insulin, lower levels of hyperinsulinemia are obtained, e.g., systemic insulin concentrations are

at least about 20% lower when compared to a comparably effective subcutaneous dose of insulin. Therefore, it is believed that the present invention provides a method for reducing the incidence and/or severity of systemic hyperinsulinemia associated with chronic dosing of insulin, and it is believed that the present invention also provides a method for reducing the incidence and/or severity of one or more disease states associated with chronic dosing of insulin.

By virtue of the chronic administration of oral dosage forms of the present invention, it is believed that the patient achieves improved glucose tolerance and glycemic control as compared with baseline levels prior to treatment, even without any statistically significant increase in weight, risk of hypoglycemia or risk of hyperinsulinemia over the treatment period. Further, by virtue of the chronic administration of oral dosage forms of the present invention, it is believed that the patient achieves improved insulin utilization, insulin sensitivity insulin secretion capacity and $HbA_1c$ levels as compared with baseline levels prior to treatment.

It is also believed that the chronic administration of oral dosage forms of the present invention to replace the endogenous insulin production in a mammal with impaired glucose tolerance or early stage diabetes mellitus will result in prophylactically sparing the function of the mammal's β-cells or will prevent death or dysfunction of the mammal's β-cells, and will thereby provide long-term protection to the mammal from developing overt or insulin dependent diabetes, or will delay the onset of overt or insulin dependent diabetes in the mammal.

The preferred pharmaceutical compositions of the invention comprise a combination of insulin and a biguanide in a suitable pharmaceutical carrier or excipient as understood by practitioners in the art. Further preferred pharmaceutical compositions of the invention additionally comprise a delivery agent that facilitates the absorption of insulin from the gastrointestinal tract. The means of delivery of the pharmaceutical composition can be, for example, a capsule, compressed tablet, pill, solution, freeze-dried, powder ready for reconstitution or suspension suitable for administration to the subject.

As used herein, "insulin" refers to insulin from a variety of sources, including naturally occurring or derived insulin, recombinant insulin, such as human and non-human recombinant insulin. Naturally occurring insulin and structurally similar bioactive equivalents (insulin analogues including short acting and analogues with protracted action) can be used. Insulin useful in the invention can be may be obtained by isolating it from natural source, such as different species of mammal. For example, animal insulin preparations extracted from bovine or porcine pancreas can be used. Insulin analogues, fragments, mimetics or polyethylene glycol (PEG)-modified derivatives of these compounds, derivatives and bioequivalents thereof can also be used with the invention.

The insulin used in the present invention may be obtained by chemically synthesizing it using protein chemistry techniques such as peptide synthesis, or by using the techniques of molecular biology to produce recombinant insulin in bacteria or eukaryotic cells. The physical form of insulin may include crystalline and/or amorphous solid forms. In addition, dissolved insulin may be used. Other suitable forms of insulin, including, but not limited to, synthetic forms of insulin, are described in U.S. Patents Nos. 4,421,685, 5,474,978, and 5,534,488, the disclosure of each of which is hereby incorporated by reference in its entirety.

The most preferred insulin useful in the pharmaceutical compositions and methods of the present invention is human recombinant insulin optionally having counter ions including zinc, sodium, calcium and ammonium or any combination thereof. Human recombinant insulin can be prepared using genetic engineering techniques that are well known in the art. Recombinant insulin can be produced in bacteria or eukaryotic cells. Functional equivalents of human recombinant insulin are also useful in the invention. Recombinant human insulin can be obtained from a variety of commercial sources. For example, insulin (Zinc, human recombinant) can be purchased from Calbiochem (San Diego, CA) or Diosynth, Inc. (The Netherlands). Alternatively, human recombinant Zinc-Insulin Crystals: Proinsulin Derived (Recombinant DNA Origin) USP Quality can be obtained from Eli Lilly and Company (Indianapolis, IN). All such forms of insulin, including insulin analogues (including but not limited to Insulin Lispro, Insulin Aspart, Insulin Glargine, and Insulin Detemir) are deemed for the purposes of this specification and the appended claims are considered to be encompassed by the term "insulin." The present invention also provides compositions of recombinant human zinc insulin and a delivery agent as a drug for oral administration of insulin in humans.

In other preferred embodiments of the invention, the insulin is a modified insulin, such as that conjugated with an oligomer such as that described in U.S. Patent No. 6,309,633 and/or which not has been subjected to amphiphilic modification such as that described in U.S. Patent Nos. 5,359,030; 5,438,040; and/or 5,681,811. The conjugated (modified) insulin may be incorporated into the oral formulations of the present invention in addition to or in the absence of any of the types of insulin described above, as well as with other insulin analogues. In such embodiments, the oral formulations include the modified insulin either with or without a pharmaceutically acceptable delivery agent that facilitates absorption of said insulin from the gastrointestinal tract.

The total amount of insulin to be used can be determined by those skilled in the art. It is preferable that the oral dosage form comprise a therapeutically effective amount of insulin, i.e., a pharmacologically or biologically effective amount, or an amount effective to accomplish the purpose of insulin. The dose of insulin administered should preferably be in such an amount that, upon oral administration, it results in a measurable and statistically significant reduction in blood glucose levels in normal healthy human subjects.

However, the amount can be less than a pharmacologically or biologically effective amount when the composition is used in a dosage unit form, such as a tablet, because the dosage unit form may contain a multiplicity of delivery agent/biologically or chemically active agent compositions or may contain a divided pharmacologically or biologically effective amount. The total effective amounts can then be administered in cumulative units containing, in total, pharmacologically, biologically or chemically active amounts of biologically or pharmacologically active agent.

It has been found that the use of delivery agent provides extremely efficient delivery of insulin. Preferred insulin doses contained in one or more dosage forms, when dosed in combination with the delivery agents described herein, are about 50 to about 600 insulin Units USP (from about 2 to about 23 mg), preferably from about 100 Units (3.8 mg) to about 450 Units (15.3 mg), more preferably from about 200 Units (7.66 mg) to about 350 Units (13.4 mg), and still more preferably about 300 Units (11.5 mg), based on the accepted conversion of factor of 26.11 Units per mg.

Still, other doses may be acceptable depending on the individual and the severity of the condition being treated, and considering that combinations of drugs may produce synergistic effects. For example doses of insulin per patient weight ranging from about 0.1 mg/kg to about 0.25 mg/kg are preferable.

Presently, different forms of typically subcutaneously-administered insulin preparations have been developed to provide different lengths of activity (activity profiles), often due to ingredients administered with insulin, ranging from short or rapid activity (e.g., solutions of regular, crystalline zinc insulin for injection; semilente insulin (prompt insulin zinc suspension); intermediate activity (e.g., NPH (isophane insulin suspension; lente (insulin zinc suspension; lente is a mixture of crystallized (ultralente) and amorphous (semilente) insulins in an acetate buffer); and slow activity (ultralente, which is extended insulin zinc suspension; protamine zinc). Short-acting insulin preparations that are commercially available in the U.S. include regular insulin and rapid-acting insulins. Regular insulin has an onset of action of 30-60 minutes, peak time of effect of 1.5 to 2 hours, and a duration of activity of 5 to 12 hours. Rapid acting insulins, such as aspart (Humalog®)/lispro (Novolog®), have an onset of action of 10-30 minutes, peak time of effect of 30-60 minutes, and a duration of activity of 3 to 5 hours. Intermediate-acting insulins, such as NPH (neutral protamine Hagedorn) and Lente insulins (insulin zinc suspension), have an onset of action of 1-2 hours, peak time of effect of 4 to 8 hours, and a duration of activity of 10 to 20 hours. In the case of long-acting insulins, Ultralente insulin has an onset of action of 2-4 hrs, peak time of effect of 8-20 hours, and a duration of activity of 16 to 24 hours, while Glargine insulin has an onset of action of 1 to 2 hours, a duration of action of 24 hours but no peak effect.

There are over 180 individual insulin preparations available world-wide. Approximately 25% of these are soluble insulin (unmodified form); about 35% are basal insulins (mixed with NPH or Lente insulins, increased pI, or isoelectric point (insulin glargine), or acylation (insulin detemir); these forms have reduced solubility, slow subcutaneous absorption and long duration of action relative to soluble insulins); about 2% are rapid-acting insulins (e.g., which are engineered by amino-acid change, and have reduced self-association and increased subcutaneous absorption); and about 38% pre-mixed insulins (e.g., NPH/soluble/rapid-acting insulins; these preparations have the benefit, e.g., of reduced number of daily injections). In many cases, regimens that use insulin in the management of diabetes combine long-acting and short-acting insulin.

It is contemplated that the oral insulin formulations of the present invention may be utilized in combination therapy to include an insulin that has rapid action, intermediate action, and/or slow action, as described above, in order to provide effective basal insulin levels in the diabetic patient. The rate of action of the insulin may be caused by virtue of its solubility, and/or by virtue of its half-life, etc. Thus, in alternative embodiments, the oral formulations of the present invention may be designed to provide the intermediate activity which is found with, e.g., a subcutaneously administered NPH insulin, or a slow action which is found with protamine zinc insulin. In each case, the oral formulations of the invention, which preferably include a pharmaceutically acceptable delivery agent which facilitates absorption of the insulin (as described herein) provide effective control of blood glucose levels, albeit for different time periods and with different plasma glucose time curves.

Intermediate-acting and long-acting insulin may be prepared using methodologies known to those skilled in the art to provide a continuous level of insulin, similar to the slow, steady (basal) secretion of insulin provided by the normal pancreas. For example, Lantus®, from Aventis Pharmaceuticals Inc., is a recombinant human insulin analog that is a long-acting, parenteral blood-glucose-lowering agent whose longer duration of action (up to 24 hours) is directly related to its slower rate of absorption. Lantus® is administered subcutaneously once a day, preferably at bedtime, and is said to provide a continuous level of insulin, similar to the slow, steady (basal) secretion of insulin provided by the normal pancreas. The activity of such a long-acting insulin results in a relatively constant concentration/time profile over 24 hours with no pronounced peak, thus allowing it to be administered once a day as a patient’s basal insulin. Such long-acting insulin has a long-acting effect by virtue of its chemical composition, rather than by virtue of an addition to insulin when administered.

In a preferred embodiment, administration of the pharmaceutical formulation comprising long-acting insulin is once or twice a day. In a preferred embodiment, administration of the dosage form comprising short-acting insulin can be once, twice, three times, four times or more than four times daily, and can be at nighttime, in the morning and/or preprandially. In a more preferred embodiment, administration of the dosage form is preferably at nighttime or morning

and three times preprandially, and more preferably is at nighttime and preprandially for breakfast, lunch and dinner. Preferably, the insulin formulations are administered to such human patients on a chronic basis, e.g., for at least about 2 weeks.

In other embodiments of the invention, the oral formulations include an insulin conjugated with an oligomer such as that described in U.S. Patent No. 6,309,633 and/or which not has been subjected to amphiphilic modification such as that described in U.S. Patent Nos. 5,359,030; 5,438,040; and/or 5,681,811. The conjugated (modified) insulin may be incorporated into the oral formulations of the present invention in addition to or in the absence of any of the types of insulin described above, as well as with other insulin analogues. In such embodiments, the oral formulations preferably include the modified insulin together with a pharmaceutically acceptable delivery agent which facilitates absorption of said insulin from the gastrointestinal tract.

In certain preferred embodiments of the invention, the oral formulations of the invention provide two forms of insulin having different activity rates in order to simulate the biphasic release of insulin in non-diabetic humans. For example, such oral formulations may include a rapid-acting form of insulin together with a slow acting form of insulin so as to provide a first peak of insulin which occurs rapidly and is short-lived, followed by a second peak of insulin which occurs at a later time, but which preferably has a longer duration.

In alternatively preferred embodiments of the invention, the methods of insulin administration of the invention provide two separate forms of insulin having different activity rates in order for the regimen to simulate the biphasic release of insulin in non-diabetic humans. For example, the oral formulations may include a rapid-acting form of insulin so as to provide a first peak of insulin which occurs rapidly and is short-lived. Such fast-acting effect may be provided by the delivery agent that facilitates the absorption of insulin from the gastrointestinal tract. The slow acting form of insulin provides a second peak of insulin that occurs at a later time but that preferably has a longer duration. Such slower acting insulin may be provided by a separate dosage form, which may be administered orally or subcutaneously.

In further embodiments of the invention, the oral dosage forms described herein reduce the likelihood of hypoglycemic events. Hypoglycemia usually results from a mismatch between insulin levels and degree of glycemia, e.g., when the administration of insulin and the ingestion of the meal are not timed such that the insulin peak occurs at peak glycemia, and administration of insulin shortly before a meal is more practical for a patient and is also safer, because glucose is ingested soon thereafter. The risk of hypoglycemia is lowered mainly due to the portal-physiologic route of administration of oral insulin. The liver cannot be hyperinsulinized, because, even under hyperinsulinemic condition, the uptake of glucose by the liver will be unchanged. Unlike the peripheral tissue, the pancreas will not sequester additional glucose but rather will only cease producing endogenous insulin. Second, the brief peak of insulin that results from the oral composition described herein shows that, even if insulin were to reach high peripheral levels, the peak quickly drops precipitously.

In addition, further embodiments of the oral dosage forms described herein avoid the risk of hypoglycemic events that may occur in certain short acting insulin formulations, which may, between the time of administration of insulin and the time of ingestion of the meal, contribute to a lowering of blood glucose to a level that could range from undesirable to clinically hypoglycemic. In the oral dosage forms disclosed herein, dosing closer to a meal eliminated the dip in blood glucose levels, which was precarious by itself. The effect seems to have also translated to lowering of the subsequent glucose excursion

In preferred embodiments of the dosage forms described herein, the dose of insulin is, in the absence of a delivery agent, not sufficiently absorbed when orally administered to a human patient to provide a desirable therapeutic effect but said dose provides a desirable therapeutic effect when administered to said patient by another route of administration. Previous disclosures by Emisphere Technologies, Inc. solved the problem of oral absorption of insulin by providing delivery agents that facilitate transport of insulin through the mucosa of the stomach and into the bloodstream where the insulin can perform its biological function. As a result, effective oral drug delivery methods are provided to increase the oral bioavailability and absorption of insulin, which is currently administered parenterally.

In preferred embodiments, the delivery agents used in the invention have the following structure:

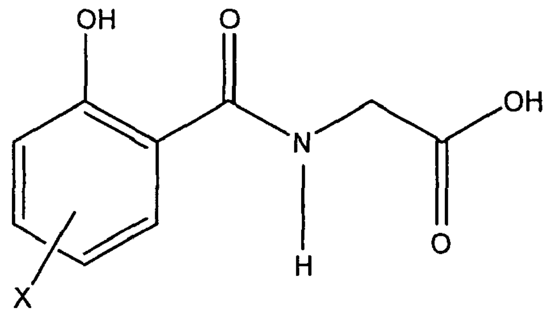

wherein X is one or more of hydrogen, halogen, hydroxyl or $C_1$-$C_3$ alkoxy, and R is substituted or unsubstituted $C_1$-$C_3$ alkylene, substituted or unsubstituted $C_1$-$C_3$ alkenylene.

In certain preferred embodiments, the delivery agents of the invention preferably have the following structure:

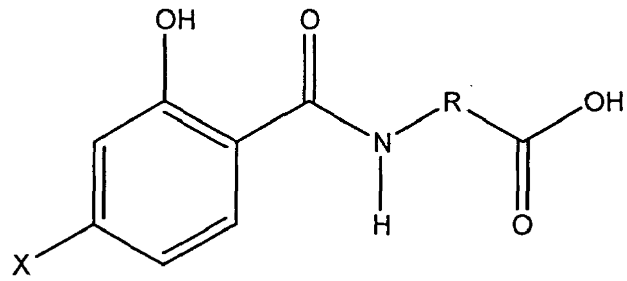

wherein X is halogen, and R is substituted or unsubstituted $C_1$-$C_3$ alkylene, substituted or unsubstituted $C_1$-$C_3$ alkenylene.

In a preferred embodiment of the present invention, the pharmaceutical composition includes a delivery agent wherein X is chlorine and R is $C_3$ alkylene. In another preferred embodiment of the present invention, the pharmaceutical composition includes the compound 4-[(4-chloro, 2-hydroxybenzoyl)amino]butanoic acid (also known as 4-[(2-hydroxy-4-chlorobenzoyl)amino]butanoate) as a delivery agent for the oral delivery of insulin, preferably the monosodium salt thereof, referred to by the term "4-CNAB". In preferred embodiments, the oral dosage forms of the present invention comprise a mixture of insulin and a delivery agent, e.g., monosodium N-(4-chlorosalicyloyl)-4-aminobutyrate (4-CNAB), a novel compound discovered by Emisphere Technologies, Inc., or separately containing insulin and the delivery agent.

The term "sodium 4-CNAB" and "mono-sodium 4-CNAB" refer to monosodium 4-[(2-hydroxy-4-chlorobenzoyl) amino]butanoate, including anhydrous, monohydrate, and isopropanol solvates thereof and amorphous and polymorphic forms thereof (including those described in International Publication No. WO 03/057650 which is hereby incorporated by reference), unless otherwise indicated. Unless otherwise noted, the term "4-CNAB" refers to all forms of 4-CNAB, including all amorphous and polymorphic forms of 4-CNAB.

The delivery agents may be in the form of the carboxylic acid or salts thereof. Suitable salts include, but are not limited to, organic and inorganic salts, for example alkali-metal salts, such as sodium, potassium and lithium; alkaline-earth metal salts, such as magnesium, calcium or barium; ammonium salts; basic amino acids, such as lysine or arginine; and organic amines, such as dimethylamine or pyridine. Preferably, the salts are sodium salts. The salts may be mono- or multivalent salts, such as monosodium salts and di-sodium salts. The salts may also be solvates, including ethanol solvates, and hydrates.

Other suitable delivery agents that can be used in the present invention include those delivery agents described United States Patents Nos. 5,650,386, 5,773,647, 5,776,888, 5,804,688, 5,866,536, 5,876,710, 5,879,681, 5,939,381, 5,955,503, 5,965,121,5,989,539, 5,990,166, 6,001,347, 6,051,561, 6,060,513, 6,090,958, 6,100,298, 5,766,633, 5,643,957, 5,863,944, 6,071,510 and 6,358,504, the disclosure of each of which is incorporated herein by reference. Additional suitable delivery agents are also described in International Publications Nos. WO 01/34114, WO 01/21073, WO 01/41985, WO 01/32130, WO 01/32596, WO 01/44199, WO 01/51454, WO 01/25704, WO 01/25679, WO 00/50386, WO 02/02509, WO 00/47188, WO 00/07979, WO 00/06534, WO 98/25589, WO 02/19969, WO 00/59863, WO 95/28838, WO 02/19969, WO 02/20466, WO 02/069937 and WO 02/070438, the disclosure of each of which is incorporated herein by reference.

Salts of the delivery agent compounds of the present invention may be prepared by methods known in the art.

For example, sodium salts may be prepared by dissolving the delivery agent compound in ethanol and adding aqueous sodium hydroxide.

The compounds described herein may be derived from amino acids and can be readily prepared from amino acids by methods known by those with skill in the art based upon the present disclosure and the methods described in International Publications Nos. WO 96/30036, WO 97/36480, WO 98/34632 and WO 00/07979, and in United States Patents Nos. 5,643,957 and 5,650,386, the disclosure of each of which is incorporated herein by reference. For example, the compounds may be prepared by reacting the single amino acid with the appropriate acylating or amine-modifying agent, which reacts with a free amino moiety present in the amino acid to form amides. Protecting groups may be used to avoid unwanted side reactions as would be known to those skilled in the art.

The delivery agents may also be prepared by the methods of International Patent Publications Nos. WO 02/02509 and WO 03/057170, the disclosure of each of which is incorporated herein by reference. The delivery agents may also be prepared by alkylation of the appropriate salicylamide according to the methods of International Publication No. WO 00/46182, the disclosure of which is incorporated herein by reference. The salicylamide may be prepared from salicylic acid via the ester by reaction with sulfuric acid and ammonia.

In addition, polyamino acids and peptides comprising one or more of these compounds may be used. An amino acid is any carboxylic acid having at least one free amine group and includes naturally occurring and synthetic amino acids. Poly amino acids are either peptides (which are two or more amino acids joined by a peptide bond) or are two or more amino acids linked by a bond formed by other groups which can be linked by, e.g., an ester or an anhydride linkage. Peptides can vary in length from dipeptides with two amino acids to polypeptides with several hundred amino acids.

The delivery agent compound may be purified by recrystallization or by fractionation on one or more solid chromatographic supports, alone or linked in tandem. Suitable recrystallization solvent systems include, but are not limited to, ethanol, water, heptane, ethyl acetate, acetonitrile, methanol and tetrahydrofuran and mixtures thereof. Fractionation may be performed on a suitable chromatographic support such as alumina, using methanol/n-propanol mixtures as the mobile phase; reverse phase chromatography using trifluoroacetic acid/ acetonitrile mixtures as the mobile phase; and ion exchange chromatography using water or an appropriate buffer as the mobile phase. When anion exchange chromatography is performed, preferably a 0-500 mM sodium chloride gradient is employed.

Following oral administration of the pharmaceutical compositions of the present invention, the delivery agent passes though the mucosal barriers of the GI tract and is absorbed into the blood stream where it can be detected in the plasma of subjects. The delivery agent facilitates the absorption of the insulin administered therewith (either in the same dosage form, or simultaneously therewith), or sequentially (in either order, as long as both the delivery agent and the insulin are administered within a time period which provides both in the same location, e.g., the stomach, at the same time). The mechanism by which 4-CNAB facilitates the gastrointestinal absorption of insulin has not yet been fully elucidated. The current working hypothesis is that 4-CNAB interacts with insulin non-covalently, creating more favorable physicochemical properties for absorption. This working hypothesis is provided for explanation purposes only and is not intended to limit the present invention or the appended claims in any way.

The amount of delivery agent in the present composition is a delivery effective amount and can be determined for any particular delivery agent/insulin combination by methods known to those skilled in the art. The amount of delivery agent necessary to adequately deliver the therapeutic amount of insulin into the blood stream of a subject needing the therapeutic effect of insulin may vary depending on one or more of the following; the chemical nature of insulin; the chemical structure of the particular delivery agent; the nature and extent of interaction between insulin and delivery agent; the nature of the unit dose, i.e., solid, liquid, tablet, capsule or suspension; the concentration of delivery agent in the GI tract; the feeding state of the subject; the diet of the subject; the health of the subject and the ratio of delivery agent to insulin. In certain preferred embodiments of the invention where the oral pharmaceutical composition includes insulin, the amount of the delivery agent preferred for the pharmaceutical composition and contained in one or more dosage forms is from about 1 mg to about 2,000 mg, more preferably from about 5 mg to about 800 mg, more preferably about 20 mg to about 600 mg, even more preferably from about 30 mg to about 400 mg, still more preferably from about 40 mg to about 200 mg, most preferably about 40 mg, 80 mg or 160 mg.

The time it takes for the delivery agent to reach a peak in the bloodstream ($t_{max}$) may depend on many factors such as the following: the nature of the unit dose, i.e., solid, liquid, tablet, capsule, suspension; the concentration of delivery agent in the GI tract; the feeding state of the subject; the diet of the subject; the health of the subject and the ratio of delivery agent to the active agent. The delivery agents of the present invention are rapidly absorbed from the gastrointestinal tract when orally administered in an immediate release dosage form, preferably in tablet form, and preferably provide a peak plasma delivery agent concentration within about 5 minutes to about 40 minutes after oral administration, and preferably at about 10 minutes to about 35 minutes after oral administration. In a preferred embodiment of the invention, wherein the pharmaceutical composition includes the compound 4-CNAB as the delivery agent for insulin, the composition provides a peak plasma delivery agent concentration within about 25 minutes to about 35 minutes after oral administration to fasting diabetic patients and within about 15 minutes to about 25 minutes after oral administration to fed diabetic patients.

In certain preferred embodiments of the invention, a peak plasma concentration ($C_{max}$) of the delivery agent achieved after oral administration is preferably from about 10 to about 250,000 ng/ml, after oral administration, preferably from about 100 to about 125,000 ng/ml, and preferably the peak plasma concentration of the delivery agent is from about 1,000 to about 50,000 ng/ml, after oral administration. More preferably, the peak plasma concentration of the delivery agents of the present invention is from about 3,000 to about 15,000 ng/ml after oral administration.

Thus, in certain preferred embodiments of the present invention, the oral insulin formulations of the invention may be administered to a patient at meal time, and preferably slightly before (e.g., about 10-30 minutes before) ingestion of a meal, such that the peak plasma insulin concentrations are attained at or about the time of peak blood glucose concentrations resulting from the meal. As a further advantage in certain preferred embodiments, the administration of a relatively short-acting insulin will further result in plasma insulin levels returning to baseline levels within about 4 hours (and preferably within about 3 hours or less) after oral administration of the insulin formulations of the present invention.

In a preferred embodiment of the invention, wherein the pharmaceutical composition includes the compound 4-CNAB as the delivery agent and insulin as the active agent, the composition provides a peak plasma 4-CNAB concentration within about 0.1 to about 3 hours after oral administration. In certain preferred embodiments where the pharmaceutical composition includes the compound 4-CNAB as the delivery agent and insulin as the active agent, the peak plasma concentration of delivery agent attained is from about 8,000 to about 37,000 ng/ml.

The optimum ratio of insulin to delivery agent can vary depending on the delivery agent and the formulation. Optimizing the ratio of insulin to delivery agent is within the knowledge of one skilled in the art. In certain preferred embodiments of the invention, the pharmaceutical composition includes insulin as the active agent and the delivery agent is the monosodium salt of 4-CNAB, the ratio of insulin [Units] to delivery agent [mg] ranges from 10:1 [Units/mg] to 1:10 [Units/mg], preferably, the ratio of insulin [Units] to delivery agent [mg] ranges from 5:1 [Units/mg] to 0.5:1 [Units/mg].

Absorption of insulin can be detected in subjects treated with the pharmaceutical compositions of the present invention by monitoring the plasma levels of insulin after treatment. The time it takes for an active agent to reach a peak in the bloodstream ($t_{max}$) may depend on many factors such as the following: the nature of the unit dose, i.e., solid, liquid, tablet, capsule, suspension; the concentration of active agent and delivery agent in the GI tract; the feeding state of the subject; the diet of the subject; the health of the subject and the ratio of active agent to the delivery agent.

In a preferred embodiment of the invention, wherein the pharmaceutical composition comprises the compound 4-CNAB as the delivery agent and insulin as the active agent, the composition provides a peak plasma insulin concentration from about 0.1 to about 1 hour after oral administration. In another embodiment, the composition provides a peak plasma insulin concentration from about 0.2 to about 0.6 hours after oral administration. In a preferred embodiment, the composition provides a peak plasma insulin concentration from about 0.3 to about 0.4 hours after oral administration. In another embodiment, the composition provides a peak plasma insulin concentration within about 1 hour after oral administration. In certain preferred embodiments, the pharmaceutical composition comprises insulin and the compound 4-CNAB as a delivery agent to facilitate the oral delivery of insulin, and after insulin is absorbed into the bloodstream, the plasma insulin levels in treated patients peak at from about 10 to about 20 minutes post oral administration with a second peak at about 105 minutes.

The effect of absorption of insulin is manifested in human patients treated with the pharmaceutical compositions of the present invention by observing reductions in C-peptide concentration following oral treatment. For example, in one embodiment of the invention, the pharmaceutical composition comprises insulin and the compound 4-CNAB as a delivery agent to facilitate the oral delivery of insulin, and, after insulin is absorbed into the bloodstream, the composition produces a maximal decrease in C-peptide concentration in treated patients from about 80 and about 120 minutes post oral administration. More particularly, the composition produces a maximal decrease in C-peptide concentration in treated patients from about 90 and about 110 minutes post oral administration.

In previous patent applications, such as those enumerated above that have been incorporated herein by reference, Emisphere Technologies, Inc. disclosed structures of various delivery agents, comparisons of their effectiveness of absorption and effectiveness of delivery, the preparation of the preferred delivery agent 4-CNAB, its preparation for human studies, and data regarding previous non-clinical and clinical studies involving the delivery agent 4-CNAB.

In a preferred embodiment of the present invention, the oral pharmaceutical dosage form described herein includes an additional drug, in particular an oral hypoglycemic agent, namely one or more of the various types of biguanides mentioned above, in addition to a type of insulin and a delivery agent as described above.

In preferred embodiments of the invention, the pharmaceutical dosage form of the invention includes one of the biguanides, for example phenformin, buformin or metformin, such as 1,1-Dimethylbiguanidine. Two drugs from the biguanide class, metformin and phenformin, were developed in 1957. Unfortunately, while phenformin resulted in several deaths from lactic acidosis and was pulled from drugstore shelves worldwide, metformin was eventually found to be 20 times less likely to cause lactic acidosis. Metformin first became available in France in 1979, and was cleared for therapeutic use for Type 2 diabetes in the U.S. in 1994. Metformin, specifically metformin hydrochloride in tablet form (Glucophage® and Glucophage® XR by Bristol-Myers Squibb Company of Princeton, NJ), is currently the only biguanide

available for therapeutic use.

Metformin is a chemical kin to the French lilac plant, which was noted in the early 1900's to lower the blood sugar. However, French lilac, like phenformin, turned out to be too toxic for use in humans. Metformin, with a much shorter action time than phenformin, has a much lower risk for severe side effects and is quite safe for use by anyone who is otherwise healthy. In fact, in the major UKPDS study, it was the only drug that reduced diabetes-related death rates, heart attacks, and strokes. However, it should not be used by those who use more than two ounces or two drinks of alcohol a day, who have congestive heart failure, or who have significant kidney, liver, or lung disease. Metformin hydrochloride is a white to off-white crystalline compound with a molecular formula of $C_4H_{11}N_5$ • HCl and a molecular weight of 165.63. Metformin hydrochloride is freely soluble in water and is practically insoluble in acetone, ether and chloroform. The $pK_a$ of metformin is 12.4, and the pH of a 1% aqueous solution of metformin hydrochloride is 6.68.

Metformin decreases hepatic glucose production by inhibiting gluconeogenesis and glycogenolysis, decreases (or delays) intestinal absorption of glucose and improves insulin sensitivity by increasing peripheral glucose uptake and utilization. Metformin lowers fasting blood glucose levels by an average of 25% (17 to 37%), postprandial blood glucose up to 44.5%, and the A1c by an average of 1.5% (0.8 to 3.1%). Metformin reduces raised plasma insulin levels in cases of metabolic syndrome by as much as 30% and reduces the need for injected insulin in Type 2 diabetics by 15 to 32%. Metformin has a mean bioavailability of 50-60%. It is eliminated primarily by renal filtration and secretion and has a half-life of approximately 6 hours in patients with type 2 diabetes (its half-life is prolonged in patients with renal impairment).

Metformin possesses some distinct advantages in treating diabetes. Metformin reduces the overproduction of glucose by the liver that is the major source of high blood sugars in Type 2 diabetes and is typically the reason for high blood sugars on waking in the morning. It helps in lowering the blood sugar, especially after eating, with no risk of hypoglycemia when used alone. Metformin also has favorable effects on lipid metabolism, and this has been shown at therapeutic doses in controlled, medium-term or long term clinical studies: metformin reduces total cholesterol, LDL cholesterol and triglyceride levels. The 10 year UKPDS Study of over 3,000 people with Type 2 diabetes found that those who were placed on metformin had a 36% decrease in overall mortality and a 39% decrease in heart attacks.

Because metformin shuts off the liver's excess production of glucose, it reduces the amount of injected insulin needed to control the blood sugar in both Type 1 and Type 2 diabetes. People with Type 2 diabetes who are on insulin usually are advised to lower their insulin doses prior to starting metformin. The full improvement in glycemic control and cholesterol levels may not be seen until 4 to 6 weeks of use have passed.

Side effects from metformin include a change in taste, loss of appetite, nausea or vomiting, abdominal bloating or gas, diarrhea or skin rash, all of which may occur during the first few weeks of taking the medication but are seldom long-lasting. Lactic acidosis, the serious but rare side effect originally seen with phenformin, results when a buildup of lactic acid occurs due to an inability to clear metformin from the system. Lactic acidosis occurs very rarely, only once in every 30,000 person-years of use, and almost always occurs in older people who have another major health problem, especially one that may impair breathing or circulation, with a mortality rate of about 40%.

Metformin does not help patients who have insulin-dependent or type 1 diabetes because they cannot produce insulin from their pancreas. Because it has no effect in the absence of insulin, metformin tablets have for that reason been uniquely qualified for combination with insulin.

Metformin has also previously been used in combination therapy with other oral antidiabetic agents. For example, doctors have often prescribed both metformin and a sulfonylurea (such as glyburide or glipizide) together in the treatment of Type 2 diabetes due to the drugs' additive effects on reducing blood sugar levels and $HbA_{1C}$, and two products that combine a sulfonylurea and metformin in one tablet are available: Metaglip™ (glipizide and metformin hydrochloride) and Glucovance® (glyburide and metformin hydrochloride), both by Bristol-Myers Squibb Company. In addition, a tablet combining metformin and thiazolidinedione (rosiglitazone maleate) is available as Avandamet® by GlaxoSmithKline for treatment of type 2 diabetes. Furthermore, a recently conducted study concluded that triple therapy of metformin, thiazolidinedione and insulin improved glycemic control in type 2 diabetic patients and reduced the necessary insulin dose without increasing weight gain, as reported in Strowig SM, Aviles-Santa ML, Raskin P, Diabetes Care, Vol. 27, No. 7, pages 1577-83 (July 2004).

Suitable unit dosages of the biguanide hypoglycemic agent, such as metformin, include the known doses for these compounds as described or referred to in reference texts such as the British and U.S. Pharmacopoeias, Remington's Pharmaceutical Sciences (Mack Publishing Co.), Martindale The Extra Pharmacopoeia (London, The Pharmaceutical Press) (for example see the 31st Edition, page 341 and pages cited therein) or the above mentioned publications.

There is no fixed dosage of Glucophage® for the management of hyperglycemia, and dosage must be individualized based upon effectiveness and tolerance, while not exceeding the maximum recommended daily dose of 2550 mg in adults and 2000 mg in pediatric patients, once or in divided doses. In general, clinically significant results are not seen at doses below 1500 mg per day. However, a lower recommended starting dose and gradually increased dosage is advised in order to minimize gastrointestinal symptoms. The maximum recommended daily dose of metformin is 3 g, taken in three doses with meals.

Suitable dosages of metformin include up to 3000 mg per day, in unit doses of 500 mg (for example, two or

three times per day) to 850 mg (for example, two times per day), depending on the clinical needs of the patient. One example of a dosage for metformin is 500 mg once per day, building to five times per day. Thus, a daily dose may be contained in one dosage form of the invention or may be contained in more than one such dosage form.

At the usual metformin doses and dosing schedules, steady state plasma concentrations are reached within 24 to 48 hours and are generally less than 1 μg/mL. In controlled clinical trials, maximum metformin plasma levels ($C_{max}$) did not exceed 4 μg/mL, even at maximum doses. Absolute bioavailability of a 500 mg or 850 mg metformin tablet is approximately 50-60% in healthy subjects. After an oral dose, the non-absorbed fraction recovered in feces was 20-30%.

In certain embodiments of the present invention, the composition provides a mean $C_{max}$ of metformin from about 500 to about 700 ng/ml. In certain embodiments, the composition provides a mean $t_{max}$ at from about 2 to about 3 hours after oral administration.

The delivery agent and biguanide may be mixing directly with the unmodified insulin prior to administration, either in dry powder form or wet granulated together. To this mixture, other pharmaceutically acceptable excipients may be added. The mixture may be then tableted or placed into gelatin capsules. Alternatively, the delivery agent/insulin mixture may be prepared as an oral solution or suspension. The delivery agent, insulin and biguanide do not need to be mixed together prior to administration, such that, in certain embodiments, the unit dose of insulin (with or without other pharmaceutically acceptable excipients), the delivery agent (with or without other pharmaceutically acceptable excipients) and the biguanide (with or without other pharmaceutically acceptable excipients) are separately orally administered separately, sequentially or simultaneously.

In certain preferred embodiments, the oral dosage forms of the present invention are solid. The insulin in dry powder form is stable, and in certain preferred embodiments is simply mixed in desirable ratios with the delivery agent and the biguanide. The dry powder mixture may then be filled into gelatin capsules, with or without optional pharmaceutical excipients. Alternatively, the insulin in dry powder form may be mixed with the delivery agent and the biguanide together with optional pharmaceutical excipients and additives such as phosphate buffer salts, citric acid, acetic acid, gelatin, and gum acacia, and the mixture may be tableted in accordance with standard tableting procedures known to those having ordinary skill in the art.

The dosage forms of the present invention may be produced by first dissolving insulin, the delivery agent and the biguanide into one solution or separate solutions. The solvent will preferably be an aqueous solution, but organic solvents or aqueous organic solvent mixtures may be used when necessary to solubilize the delivery agent. If two solutions are used, the proportions of each necessary to provide the correct amount of insulin, delivery agent or the biguanide are combined and the resulting solution may be dried, by lyophilization or equivalent means. In one embodiment of the invention, the oral dosage form may be dried and rehydrated prior to oral administration.

In preferred embodiments of the oral dosage forms of the invention described above, the oral dosage form is solid, and is preferably provided incorporated within a gelatin capsule or is contained in a tablet.

Stabilizing additives may be incorporated into the delivery agent solution. With some drugs, the presence of such additives promotes the stability and dispersibility of the agent in solution. The stabilizing additives may be employed at a concentration ranging from about 0.1 and 5% (W/V), preferably about 0.5% (W/V). Suitable, but non-limiting, examples of stabilizing additives include gum acacia, gelatin, methyl cellulose, polyethylene glycol, carboxylic acids and salts thereof, and polylysine. The preferred stabilizing additives are gum acacia, gelatin and methyl cellulose.

The oral dosage forms of the present invention, containing a mixture of the active agent, e.g., insulin and the delivery agent, e.g., 4-CNAB or separately containing the active agent and the delivery agent, may include additional materials known to those skilled in the art as pharmaceutical excipients. Any excipient or ingredient, including pharmaceutical ingredients or excipients. Such pharmaceutical excipients include, for example, the following: Acidifying agents (acetic acid, glacial acetic acid, citric acid, fumaric acid, hydrochloric acid, diluted hydrochloric acid, malic acid, nitric acid, phosphoric acid, diluted phosphoric acid, sulfuric acid, tartaric acid); Aerosol propellants (butane, dichlorodifluoromethane, dichlorotetrafluoroethane, isobutane, propane, trichloromonofluoromethane); Air displacements (carbon dioxide, nitrogen); Alcohol denaturants (denatonium benzoate, methyl isobutyl ketone, sucrose octacetate); Alkalizing agents (strong ammonia solution, ammonium carbonate, diethanolamine, diisopropanolamine, potassium hydroxide, sodium bicarbonate, sodium borate, sodium carbonate, sodium hydroxide, trolamine); Anticaking agents (see *glidant*); Antifoaming agents (dimethicone, simethicone); Antimicrobial preservatives (benzalkonium chloride, benzalkonium chloride solution, benzelthonium chloride, benzoic acid, benzyl alcohol, butylparaben, cetylpyridinium chloride, chlorobutanol, chlorocresol, cresol, dehydroacetic acid, ethylparaben, methylparaben, methylparaben sodium, phenol, phenylethyl alcohol, phenylmercuric acetate, phenylmercuric nitrate, potassium benzoate, potassium sorbate, propylparaben, propylparaben sodium, sodium benzoate, sodium dehydroacetate, sodium propionate, sorbic acid, thimerosal, thymol); Antioxidants (ascorbic acid, acorbyl palmitate, butylated hydroxyanisole, butylated hydroxytoluene, hypophosphorous acid, monothioglycerol, propyl gallate, sodium formaldehyde sulfoxylate, sodium metabisulfite, sodium thiosulfate, sulfur dioxide, tocopherol, tocopherols excipient); Buffering agents (acetic acid, ammonium carbonate, ammonium phosphate, boric acid, citric acid, lactic acid, phosphoric acid, potassium citrate, potassium metaphosphate, potassium phosphate monobasic, sodium acetate, sodium citrate, sodium lactate solution, dibasic sodium phosphate, monobasic sodium phosphate);

Capsule lubricants (see *tablet and capsule lubricant*); Chelating agents (edetate disodium, ethylenediaminetetraacetic acid and salts, edetic acid); Coating agents (sodium carboxymethylcellulose, cellulose acetate, cellulose acetate phthalate, ethylcellulose, gelatin, pharmaceutical glaze, hydroxypropyl cellulose, hydroxypropyl methylcellulose, hydroxypropyl methylcellulose phthalate, methacrylic acid copolymer, methylcellulose, polyethylene glycol, polyvinyl acetate phthalate, shellac, sucrose, titanium dioxide, carnauba wax, microcystalline wax, zein); Colorants (caramel, red, yellow, black or blends, ferric oxide); Complexing agents (ethylenediaminetetraacetic acid and salts (EDTA), edetic acid, gentisic acid ethanolmaide, oxyquinoline sulfate); Desiccants (calcium chloride, calcium sulfate, silicon dioxide); Emulsifying and/or solubilizing agents (acacia, cholesterol, diethanolamine (adjunct), glyceryl monostearate, lanolin alcohols, lecithin, mono- and di-glycerides, monoethanolamine (adjunct), oleic acid (adjunct), oleyl alcohol (stabilizer), poloxamer, polyoxyethylene 50 stearate, polyoxyl 35 caster oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, propylene glycol diacetate, propylene glycol monostearate, sodium lauryl sulfate, sodium stearate, sorbitan monolaurate, soritan monooleate, sorbitan monopalmitate, sorbitan monostearate, stearic acid, trolamine, emulsifying wax); Filtering aids (powdered cellulose, purified siliceous earth); Flavors and perfumes (anethole, benzaldehyde, ethyl vanillin, menthol, methyl salicylate, monosodium glutamate, orange flower oil, peppermint, peppermint oil, peppermint spirit, rose oil, stronger rose water, thymol, tolu balsam tincture, vanilla, vanilla tincture, vanillin); Glidants and/or anticaking agents (calcium silicate, magnesium silicate, colloidal silicon dioxide, talc); Humectants (glycerin, hexylene glycol, propylene glycol, sorbitol); Plasticizers (castor oil, diacetylated monoglycerides, diethyl phthalate, glycerin, mono- and di-acetylated monoglycerides, polyethylene glycol, propylene glycol, triacetin, triethyl citrate); Polymers (e.g., cellulose acetate, alkyl celloloses, hydroxyalkylcelloloses, acrylic polymers and copolymers); Solvents (acetone, alcohol, diluted alcohol, amylene hydrate, benzyl benzoate, butyl alcohol, carbon tetrachloride, chloroform, corn oil, cottonseed oil, ethyl acetate, glycerin, hexylene glycol, isopropyl alcohol, methyl alcohol, methylene chloride, methyl isobutyl ketone, mineral oil, peanut oil, polyethylene glycol, propylene carbonate, propylene glycol, sesame oil, water for injection, sterile water for injection, sterile water for irrigation, purified water); Sorbents (powdered cellulose, charcoal, purified siliceous earth); Carbon dioxide sorbents (barium hydroxide lime, soda lime); Stiffening agents (hydrogenated castor oil, cetostearyl alcohol, cetyl alcohol, cetyl esters wax, hard fat, paraffin, polyethylene excipient, stearyl alcohol, emulsifying wax, white wax, yellow wax); Suspending and/or viscosity-increasing agents (acacia, agar, alginic acid, aluminum monostearate, bentonite, purified bentonite, magma bentonite, carbomer 934p, carboxymethylcellulose calcium, carboxymethylcellulose sodium, carboxymethycellulose sodium 12, carrageenan, microcrystalline and carboxymethylcellulose sodium cellulose, dextrin, gelatin, guar gum, hydroxyethyl cellulose, hydroxypropyl cellulose, hydroxypropyl methylcellulose, magnesium aluminum silicate, methylcellulose, pectin, polyethylene oxide, polyvinyl alcohol, povidone, propylene glycol alginate, silicon dioxide, colloidal silicon dioxide, sodium alginate, tragacanth, xanthan gum); Sweetening agents (aspartame, dextrates, dextrose, excipient dextrose, fructose, mannitol, saccharin, calcium saccharin, sodium saccharin, sorbitol, solution sorbitol, sucrose, compressible sugar, confectioner's sugar, syrup); Tablet binders (acacia, alginic acid, sodium carboxymethylcellulose, microcrystalline cellulose, dextrin, ethylcellulose, gelatin, liquid glucose, guar gum, hydroxypropyl methylcellulose, methycellulose, polyethylene oxide, povidone, pregelatinized starch, syrup); Tablet and/or capsule diluents (calcium carbonate, dibasic calcium phosphate, tribasic calcium phosphate, calcium sulfate, microcrystalline cellulose, powdered cellulose, dextrates, dextrin, dextrose excipient, fructose, kaolin, lactose, mannitol, sorbitol, starch, pregelatinized starch, sucrose, compressible sugar, confectioner=s sugar); Tablet disintegrants (alginic acid, microcrystalline cellulose, croscarmellose sodium, corspovidone, polacrilin potassium, sodium starch glycolate, starch, pregelatinized starch); Tablet and/or capsule lubricants (calcium stearate, glyceryl behenate, magnesium stearate, light mineral oil, polyethylene glycol, sodium stearyl fumarate, stearic acid, purified stearic acid, talc, hydrogenated vegetable oil, zinc stearate); Tonicity agent (dextrose, glycerin, mannitol, potassium chloride, sodium chloride); Vehicle: flavored and/or sweetened (aromatic elixir, compound benzaldehyde elixir, iso-alcoholic elixir, peppermint water, sorbitol solution, syrup, tolu balsam syrup); Vehicle: oleaginous (almond oil, corn oil, cottonseed oil, ethyl oleate, isopropyl myristate, isopropyl palmitate, mineral oil, light mineral oil, myristyl alcohol, octyldodecanol, olive oil, peanut oil, persic oil, seame oil, soybean oil, squalane); Vehicle: solid carrier (sugar spheres); Vehicle: sterile (bacteriostatic water for injection, bacteriostatic sodium chloride injection); Viscosity-increasing (see *suspending agent*); Water repelling agent (cyclomethicone, dimethicone, simethicone); and Wetting and/or solubilizing agent (benzalkonium chloride, benzethonium chloride, cetylpyridinium chloride, docusate sodium, nonoxynol 9, nonoxynol 10, octoxynol 9, poloxamer, polyoxyl 35 castor oil, polyoxyl 40, hydrogenated castor oil, polyoxyl 50 stearate, polyoxyl 10 oleyl ether, polyoxyl 20, cetostearyl ether, polyoxyl 40 stearate, polysorbate 20, polysorbate 40, polysorbate 60, polysorbate 80, sodium lauryl sulfate, sorbitan monolaureate, sorbitan monooleate, sorbitan monopalmitate, sorbitan monostearate, tyloxapol). This list is not meant to be exclusive, but instead merely representative of the classes of excipients and the particular excipients which may be used in oral dosage forms of the present invention.

The amount of the doses of insulin, delivery agent and biguanide may depend on the individual and the severity of the condition being treated, and the fact that the combination of the drugs may produce synergistic effects. For example, doses from about 0.1 mg/kg to about 0.25 mg/kg of insulin may be combined with about 450 mg/kg of Metformin and

200 mg/kg of 4-CNAB.

Following administration, the insulin present in the dosage unit form is absorbed into the circulation. The circulating levels of the insulin itself can be measured directly. Similarly, levels of 4-CNAB delivery agent in the blood can be measured. The bioavailability of the insulin is readily assessed by measuring a known pharmacological activity in blood, e.g., decreased blood glucose. Further physiologic effects of the insulin can be measured using tests, for example, measurement of plasma C-peptide concentration as a measure of endogenous insulin production.

In addition, a fructosamine assay can be performed to determine the measure of the diabetic patient's glycemic control over the previous period of two to three weeks. Fructosamine is formed by a non-enzymatic reaction between glucose and amino acid residues of proteins, and serum fructosamine levels are elevated in diabetic patients with elevated blood glucose concentration. Whereas blood glucose concentration is a short-term indicator of diabetes control, fructosamine is a short- to medium-term indicator of diabetes control that correlates well with both fasting and mean blood glucose over a 2-week period.

In the present invention, the methods for treating a mammal with impaired glucose tolerance or with early or late stage diabetes comprise orally administering to the mammal a pharmaceutical formulation that includes a therapeutically effective amount of insulin or an insulin analog, a delivery agent in an amount effective to facilitate the absorption of the insulin from the gastrointestinal tract and a biguanide such as metformin. It is preferred that the administration be on a chronic basis, e.g., for at least two weeks, and be preprandially and at bedtime such that, after two weeks of treatment, the mammal achieves improved glucose tolerance and glycemic control, as well as improved insulin utilization, insulin sensitivity, insulin secretion capacity and $HbA_1c$ levels, as compared with baseline levels prior to treatment.

Improved glucose tolerance can be demonstrated by better endogenous capacity of the mammal to handle sugar load as measured by blood glucose concentration, following a sugar load, that is reduced by a statistically significant amount as compared with baseline blood glucose concentration, following a glucose load, prior to treatment.

Improved glucose tolerance and better endogenous capacity of the mammal to handle sugar load can also be measured by an AUC of blood glucose excursion, following a glucose load, that is reduced by a statistically significant amount as compared with AUC of blood glucose excursion, following a glucose load, prior to treatment.

Improved glycemic control can be demonstrated by:

- decreased fasting blood glucose levels as measured by fasting blood glucose concentration that is reduced by a statistically significant amount as compared with baseline fasting blood glucose concentration prior to treatment.
- decreased serum fructosamine concentrations, as measured by serum fructosamine assay, that is reduced by a statistically significant amount as compared with baseline serum fructosamine concentrations prior to treatment.
- improved HbA1c levels after treatment compared with baseline levels prior to treatment. Preferably, the improved HbA1c levels are measured by a statistically significant decline in HbA1c levels. When treating a mammal with impaired glucose tolerance or with early or late stage diabetes, administration of the pharmaceutical formulation of the present invention can preferably be made to a mammal having an $HbA_{1c}$ level ranging from normal to elevated prior to treatment.

Improved insulin utilization and insulin sensitivity of the patient's body can be measured by a statistically significant decline in HOMA (Homeostasis Model Assessment), and the improved insulin secretion capacity of the patient's body is measured by Stumvoll first-phase insulin secretion capacity index.

In preferred embodiments of the invention, by virtue of the chronic administration of oral dosage forms of the present invention, the patient achieves improved glucose tolerance and glycemic control as compared with baseline levels prior to treatment even without any statistically significant increase in weight, any statistically significant increase in risk of hypoglycemia or any statistically significant increase in risk of hyperinsulinemia in the mammal over the treatment period, and without the need for monitoring the mammal's blood glucose concentrations or $HbA_{1c}$ levels. Further, by virtue of the chronic administration of oral dosage forms of the present invention, the patient achieves improved insulin utilization, insulin sensitivity insulin secretion capacity and $HbA_{1c}$ levels as compared with baseline levels prior to treatment.

It is preferred that the administration of the oral pharmaceutical formulation will be about once daily to about four or more times daily, preprandially and/or at bedtime. In one embodiment of the invention, administration of the pharmaceutical formulation takes place once daily, either at bedtime or preprandially for one meal during the day time, e.g., for breakfast, lunch or dinner. In another embodiment, administration of the pharmaceutical formulation takes place multiple times daily, preferably at bedtime and preprandially for one meal during the day time, e.g., for breakfast, lunch or dinner. In a further embodiment, administration of the pharmaceutical formulation takes place multiple times daily, preferably at bedtime and preprandially for more than one meal during the day time. Administration of the pharmaceutical formulation can also be is at or shortly prior to bedtime and concurrently with or shortly prior to ingestion of each meal, i.e., within about 15 minutes or less of ingestion of each meal.

Preferably, the insulin formulations are administered to such human patients on a chronic basis, e.g., for at

least about two weeks. The dosage form of the present invention can be administered for at least one day, for one week, for two weeks, for longer periods, for alternating on-off time periods, or for the life of the patient.

It is believed that the frequency of administration of the oral pharmaceutical formulation, on a daily basis (i.e., how often during one day-night period) and on a chronic basis (i.e., for how many days), will depend upon the patient's position along a "diabetes continuum", i.e., the extent of the patient's impaired glucose tolerance, the patient's stage of diabetes and the patient's need for exogenous glycemic control. This continuum ranges from normal glycemic control, to simple impaired glucose tolerance and insulin resistance seen in pre-diabetics or early stage type 2 diabetics, to failure of insulin production by the pancreas seen in type 1 diabetics and late stage type 2 diabetics. This can also be measured by the patient's $HbA_1c$ concentration, ranging from normal to elevated levels.

For example, if the patient has a need for fasting glycemic control, the oral pharmaceutical formulation should preferably be administered only at or shortly prior to bedtime. If the patient has some need for post-prandial glycemic control, the oral pharmaceutical formulation should preferably be administered preprandially for some meals. If the patient has a need for total post-prandial glycemic control, the oral pharmaceutical formulation should preferably be administered preprandially for all meals. If the patient has a need for comprehensive glycemic control, the oral pharmaceutical formulation should preferably be administered preprandially for all meals and at or shortly prior to bedtime.

In alternatively preferred embodiments of the invention, the additional treatment may comprise a second form of insulin, so as to provide the patient with two separate forms of insulin having different activity rates in order for the regimen to simulate the biphasic release of insulin in non-diabetic humans. For example, the oral formulations may include a rapid-acting form of insulin so as to provide a first insulin peak that occurs rapidly and is short-lived, and the fast-acting effect may be provided by the delivery agent that facilitates the absorption of insulin from the gastrointestinal tract. The slow acting form of insulin provides a second insulin peak that occurs later but has a longer duration. Such slower acting insulin may be provided by the same oral formulation as the rapid-acting insulin or by a separate dosage form that may be administered orally or subcutaneously.

It is further believed that the particular combination therapy and its frequency of administration, on a daily basis and on a chronic basis, will depend upon the patient's position along the "diabetes continuum". For example, if the patient has a need for fasting glycemic control, the oral pharmaceutical formulation should be administered only at or shortly prior to bedtime. If the patient has some need for post-prandial glycemic control, the oral pharmaceutical formulation should be administered preprandially for meals. If the patient has a need for basal insulin, as in late stage type 2 diabetes or type 1 diabetes, the supplemental slow-acting insulin or anti-diabetic drug should be administered daily. If the patient has a need for comprehensive glycemic control, the oral pharmaceutical formulation should preferably be administered preprandially for all meals and at or shortly prior to bedtime in combination with the slow-acting insulin or anti-diabetic drug.

It is also believed that the invention provides a method of achieving glucose homeostasis in mammals, comprising orally administering to a mammal a pharmaceutical formulation comprising a therapeutically effective amount of insulin or an insulin analog and a delivery agent in an amount effective to facilitate the absorption of the insulin from the gastrointestinal tract. It is preferred that the administration be on a chronic basis, e.g., for at least two weeks, and be preprandially and at bedtime such that, after two weeks of treatment, the mammal achieves improved glucose tolerance and glycemic control as compared with baseline levels prior to treatment.

It is further believed that the chronic administration of the oral dosage forms of the present invention will reduce the incidence and/or severity of systemic hyperinsulinemia associated with chronic dosing of insulin or of one or more disease states associated with chronic dosing of insulin in a mammal that has impaired glucose tolerance or early stage diabetes.

The chronic administration of oral dosage forms of the present invention result in a higher portal insulin concentration and lower systemic insulin concentration over time than that obtained with an equi-effective dose of insulin administered subcutaneously (i.e., which provide similar control of blood glucose levels). Transient peaks in insulin levels that may occur by virtue of the oral administration of insulin in accordance with the present invention are not believed to be associated with vascular diseases. By virtue of the chronic administration of oral dosage forms of the present invention instead of equi-effective subcutaneous doses of insulin, lower levels of hyperinsulinemia are obtained, e.g., systemic insulin concentrations are at least about 20% lower when compared to a comparably effective subcutaneous dose of insulin.

The present invention thus provides methods for reducing the incidence and/or severity of systemic hyperinsulinemia associated with chronic dosing of insulin, and it is believed that the present invention also provides a method for reducing the incidence and/or severity of one or more disease states associated with chronic dosing of insulin.

Such methods also comprise orally administering a therapeutically effective dose of a pharmaceutical formulation comprising insulin, a delivery agent that facilitates the absorption of the insulin from the gastrointestinal tract and a biguanide, preferably Metformin, to provide a therapeutically effective reduction and/or control in blood glucose concentration and a plasma insulin concentration that is reduced relative to the plasma insulin concentration provided by a therapeutically equivalent dose of subcutaneously injected insulin. Such methods also achieve a reduction in blood glucose concentration in human diabetic patients comparable to a subcutaneous insulin injection in those patients, while

providing a lower (e.g., 20% or greater) total exposure of insulin to the peripheral blood circulation under acute, sub-acute and chronic conditions as compared to the peripheral blood insulin exposure achieved via subcutaneous injection. The determinations of blood or insulin concentration obtained in patients who have been administered subcutaneous insulin are well known to those skilled in the art.

It is still further believed that the chronic administration of oral dosage forms of the present invention to replace the endogenous insulin production in a mammal with impaired glucose tolerance or early stage diabetes mellitus will result in prophylactically sparing the function of the mammal's β-cells or will prevent death or dysfunction of the mammal's β-cells, and will thereby provide long-term protection to the mammal from developing overt or insulin dependent diabetes, or will delay the onset of overt or insulin dependent diabetes in the mammal. The rationale for this belief is set forth in International Patent Application No. PCT/US04/06943.

In order that this invention may be better understood, the following examples are set forth. These examples are for the purpose of illustration only and are not to be construed as limiting the scope of the invention in any manner.

**EXAMPLE 1**

This example describes the manufacturing procedure for Insulin/4-CNAB/metformin tablets. Each tablet is to contain about 150 units of insulin USP (equivalent to about 5.8 mg of recombinant human insulin with an as-is potency of about 26 U/mg), about 80 mg of 4-CNAB monosodium salt and about 500 mg of metformin hydrochloride. The insulin to be used in this study will obtained from Diosynth, Inc. and will meet the specifications for Human Insulin as described in the United States Pharmacopoeia.

**Composition of formulation (theoretical, all numbers are approximate):**

| Component | Weight (mg)/ tablet |
|---|---|
| 4-CNAB, monosodium salt | 80 |
| Insulin | ~5.8 mg (150 Units) |
| Metformin hydrochloride | 500 mg |
| Povidone | 3.8 |
| Anhydrous EMCOMPRESS | 152.9 |
| Magnesium Stearate | 7.5 |
| Total | 750 |

4-CNAB, metformin hydrochloride and KOLLIDON® 90F are weighed, and KOLLIDON® 90F is dissolved in water. The amount of water used in this step is about 1-50%, preferably about 15% w/w of the amount of material used in the granulation. Insulin (obtained from Diosynth, Inc.), 4-CNAB and metformin hydrochloride are blended and charged to the 5L bowl of a Key Instruments KG-5 high shear granulator. The insulin/4-CNAB/metformin blend is then granulated using the KOLLIDON® solution, and the granulation is finished with additional water as required. Granules are dried in a vacuum oven or other suitable equipment at about 20-80°C, preferably about 50°C. Partly dried granules (about 0-10% w/w, preferably about 2-3% w/w moisture) are milled through about 0.02 inch screen using hammer mill. Drying is continued to a final moisture content of less than about 1.5% w/w.

Dried granules are then assayed for insulin, 4-CNAB and metformin hydrochloride. Based on the assay results, the amounts of excipients (Anhydrous EMCOMPRESS® and magnesium stearate) are calculated and weighed. Insulin/4-CNAB/metformin granules and anhydrous EMCOMPRESS® are blended in a V-blender for about 10-20 minutes, preferably about 15 minutes, and samples are analyzed for blend uniformity. If samples pass blend uniformity specifications, magnesium stearate is then blended for about 1-5 minutes, preferably about 3 minutes. If samples do not pass blend uniformity specifications, then the mix is blended for an additional about 1-10 minutes, preferably about 5 minutes, and the assay and analysis steps are repeated. Tablets are to be compressed on an EK-0 single station press with a hardness of about 5KP-10KP, preferably about 7KP. The resulting tablet should have a hardness of about 7.8 kP, a thickness of about 2.8 mm, a diameter of about 6.5 mm, a friability of 0.02% and a disintegration time of about 6 minutes.

The resulting tablets may be studied to determine whether they would remain within specification when stored under recommended storage conditions in order to provide evidence on how the product quality varies with time under the influence of temperature and humidity. The stability tests are to be conducted in compliance with the U.S. Federal Drug Administration current Good Manufacturing Practice Standards, 21 C.F.R. § 210 and 211, and the International

Conference on Harmonization (ICH) Guidance, ICH Q1A (R2), using qualified equipment, test methods and personnel.

Tablet samples are to be packaged in a number of closed containers that are then placed in controlled temperature and humidity chambers. For room temperature stability tests, the containers are to be stored at 25°C ± 2°C / 60% ± 5 % Relative Humidity. Samples are then drawn from these chambers at specified time intervals and tested for conformance to the product stability specifications with regard to appearance (method No. AM001v2), insulin assay (method no. AM018), 4-CNAB assay (method no. AM018), moisture (method no. USP<921>), disintegration (method no. USP <701>) and, in some cases, microbial testing (method no. USP <1111>).

**EXAMPLE 2**

This example describes the results of a study wherein solutions of insulin, 4-CNAB and Metformin were administered to Sprague Dawley rats in order tri determine the efficacy of the composition.

Dosing solutions were prepared by dissolving 4-CNAB and metformin in water. These solutions were sonicated at 35°C and the pH adjusted to 6.5 - 8.5 with sodium hydroxide. Just prior to administration in rats, insulin was added from a stock solution prepared in water (pH ~ 8.0). Final dosing solutions contained either 450 mg/mL metformin alone or 200 mg/mL 4-CNAB with 0.25 mg/kg insulin (oral insulin control), or 200 mg/mL 4-CNAB with 450 mg/mL metformin and either 0.25 or 0.1 mg/mL insulin (test groups).

Sprague Dawley rats were fasted overnight (16-24h) and were divided into five groups for oral dosing (n = 5 per group). Each rat received a single oral dose (by gavage) of dosing solution at a final dose volume of 1 mL/kg. The final dose level in rats was 200 mg/kg 4-CNAB, 450 mg/kg metformin and 0.25 or 0.1 mg/kg insulin. The study also included a control group that received an oral dose of vehicle (water) alone. Blood samples were collected by the tail clip method at 0, 15, 30, 45, 60, 120, 180, 240 and 300 minutes and blood glucose was measured immediately using a handheld glucometer. The results are presented in Tables 1, 2, and 3 below and Figures 1, 2 and 3.

**Table 1: Blood Glucose mg/dL**

| | Rat | 0 | 15 | 30 | 45 | 60 | 120 | 180 | 240 | 300 |
|---|---|---|---|---|---|---|---|---|---|---|
| Vehicle PO | 101 | 79 | 94 | 88 | 84 | 80 | 73 | 73 | 74 | 66 |
| | 102 | 82 | 91 | 83 | 80 | 80 | 74 | 67 | 65 | 62 |
| | 103 | 84 | 93 | 90 | 81 | 77 | 64 | 71 | 75 | 55 |
| | 104 | 90 | 94 | 88 | 88 | 83 | 67 | 65 | 71 | 62 |
| | 105 | 81 | 98 | 94 | 89 | 80 | 66 | 65 | 56 | 66 |
| | **Mean** | **83.200** | **94.000** | **88.600** | **84.400** | **80.000** | **68.800** | **68.200** | **68.200** | **62.200** |
| | **SD** | **4.207** | **2.550** | **3.975** | **4.037** | **2.121** | **4.438** | **3.633** | **7.855** | **4.494** |
| | | | | | | | | | | |
| Ins (0.25) + 4-CNAB (200) | 201 | 65 | 52 | 32 | 41 | 57 | 63 | 57 | 73 | 58 |
| | 202 | 76 | 46 | 38 | 55 | 68 | 61 | 62 | 69 | 60 |
| | 203 | 81 | 73 | 55 | 55 | 68 | 57 | 53 | 71 | 64 |
| | 204 | 78 | 66 | 42 | 48 | 62 | 61 | 53 | 54 | 62 |
| | 205 | 74 | 67 | 69 | 63 | 66 | 53 | 59 | 73 | 59 |
| | **Mean** | **74.800** | **60.800** | **47.200** | **52.400** | **64.200** | **59.000** | **56.800** | **68.000** | **60.600** |
| | **SD** | **6.058** | **11.300** | **14.822** | **8.295** | **4.712** | **4.000** | **3.899** | **8.000** | **2.408** |
| | | | | | | | | | | |
| Metformin (450) | 301 | 108 | 113 | 98 | 91 | 81 | 57 | 56 | 53 | 70 |
| | 302 | 88 | 86 | 85 | 77 | 74 | 64 | 53 | 56 | 58 |
| | 303 | 94 | 96 | 96 | 89 | 84 | 72 | 68 | 65 | 68 |
| | 304 | 98 | 100 | 96 | 82 | 84 | 57 | 63 | 54 | 41 |
| | 305 | 107 | 111 | 103 | 87 | 83 | 69 | 62 | 63 | 75 |
| | **Mean** | **99.000** | **101.200** | **95.600** | **85.200** | **81.200** | **63.800** | **60.400** | **58.200** | **62.400** |
| | **SD** | **8.544** | **11.122** | **6.580** | **5.675** | **4.207** | **6.834** | **5.941** | **5.450** | **13.465** |
| | | | | | | | | | | |
| Ins (0.25) + 4-CNAB (200) + Metformin (450) | 401 | 99 | 92 | 78 | 81 | 67 | 62 | 54 | 39 | 62 |

(continued)

| | Rat | 0 | 15 | 30 | 45 | 60 | 120 | 180 | 240 | 300 |
|---|---|---|---|---|---|---|---|---|---|---|
| | 402 | 86 | 80 | 74 | 64 | 59 | 65 | 68 | 63 | 65 |
| | 403 | 87 | 78 | 68 | 65 | 66 | 54 | 62 | 63 | 66 |
| | 404 | 107 | 89 | 74 | 72 | 67 | 51 | 56 | 59 | 67 |
| | 405 | 85 | 83 | 60 | 52 | 56 | 54 | 65 | 63 | 66 |
| | **Mean** | **92.800** | **84.400** | **70.800** | **66.800** | **63.000** | **57.200** | **61.000** | **57.400** | **65.200** |
| | **SD** | **9.757** | **5.941** | **7.014** | **10.710** | **5.148** | **5.975** | **5.916** | **10.431** | **1.924** |
| | | | | | | | | | | |
| Ins (0.1) + 4-CNAB (200) + Metformin (450) | 501 | 79 | 83 | 65 | 61 | 65 | 59 | 61 | 51 | 52 |
| | 502 | 76 | 80 | 75 | 65 | 58 | 53 | 45 | 50 | 40 |
| | 503 | 102 | 100 | 81 | 75 | 69 | 65 | 56 | 61 | 53 |
| | 504 | 81 | 69 | 56 | 53 | 51 | 58 | 55 | 56 | 46 |
| | 505 | 86 | 101 | 89 | 80 | 75 | 74 | 72 | 65 | 62 |
| | **Mean** | **84.800** | **86.600** | **73.200** | **66.800** | **63.600** | **61.800** | **57.800** | **56.600** | **50.600** |
| | **SD** | **10.281** | **13.722** | **13.008** | **10.826** | **9.370** | **8.044** | **9.834** | **6.427** | **8.234** |

Table 1 above shows the changes in whole blood glucose from baseline (pre-dose glucose level). As shown in Table 1, the rats that received insulin and 4-CNAB (Group 1) experienced a rapid decline in whole blood glucose (mg/dL), with a maximum drop reached by about thirty minutes. At 30 minutes, the mean blood glucose level was 47.2 mg/dL (S.D. 14.22). This was followed by a slight rise in glucose over the next 30 minutes, after which the values leveled off at approximately 60 mg/dL.

The rats receiving solely metformin (Group 2) likewise experienced a more gradual decline in whole blood glucose values from baseline, which then leveled off after about 120 minutes, also lasting into the end of the testing period. The rats receiving metformin with either 0.25 mg/kg insulin or 0.1 mg/kg with insulin and 4-CNAB (Groups 3 and 4, respectively) had a more gradual decline in whole blood glucose than the insulin alone group over the first 60 minutes, leveling off and lasting until the end of the study, about five hours.

It should be noted that although both study Groups 3 and 4 had about the same mean at 60 minutes (63.0 mg/dL S.D. 5.18 for the 0.25 mg/kg group vs. 63.6 mg/dl S.D. 9.37 for the 0.1 mg/kg group), the rat receiving the higher dose began with a baseline level of an mean of 8 points less and ended the study with a mean almost 15 points lower (65.2 S.D. 1.924 vs. 50.6 S.D. 8.234, respectively). This greater drop in whole blood glucose can be accounted for by the higher dose administered to the one rat. These results are demonstrated visually in Figure 1.

**Table 2: Percent Change in Glucose at Various Time Points**

| | Rat | 0 | 15 | 30 | 45 | 60 | 120 | 180 | 240 | 300 |
|---|---|---|---|---|---|---|---|---|---|---|
| Vehicle PO | 101 | 0.000 | 18.987 | 11.392 | 6.329 | 1.266 | -7.595 | -7.595 | -6.329 | -16.456 |
| | 102 | 0.000 | 10.976 | 1.220 | -2.439 | -2.439 | -9.756 | -18.293 | -20.732 | -24.390 |
| | 103 | 0.000 | 10.714 | 7.143 | -3.571 | -8.333 | -23.810 | -15.476 | -10.714 | -34.524 |
| | 104 | 0.000 | 4.444 | -2.222 | -2.222 | -7.778 | -25.556 | -27.778 | -21.111 | -31.111 |
| | 105 | 0.000 | 20.988 | 16.049 | 9.877 | -1.235 | -18.519 | -19.753 | -30.864 | -18.519 |
| | **Mean** | **0.000** | **13.222** | **6.716** | **1.595** | **-3.704** | **-17.047** | **-17.779** | **-17.950** | **-25.000** |
| | **SD** | **0.000** | **6.744** | **7.404** | **6.094** | **4.196** | **8.105** | **7.300** | **9.642** | **7.801** |
| | | | | | | | | | | |
| Ins (0.25) + 4-CNAB (200) | 201 | 0.000 | -20.000 | -50.769 | -36.923 | -12.308 | -3.077 | -12.308 | 12.308 | -10.769 |
| | 202 | 0.000 | -39.474 | -50.000 | -27.632 | -10.526 | -19.737 | -18.421 | -9.211 | -21.053 |
| | 203 | 0.000 | -9.877 | -32.099 | -32.099 | -16.049 | -29.630 | -34.568 | -12.346 | -20.988 |
| | 204 | 0.000 | -15.385 | -46.154 | -38.462 | -20.513 | -21.795 | -32.051 | -30.769 | -20.513 |
| | 205 | 0.000 | -9.459 | -6.757 | -14.865 | -10.811 | -28.378 | -20.270 | -1.351 | -20.270 |
| | **Mean** | **0.000** | **-18.839** | **-37.156** | **-29.996** | **-14.041** | **-20.523** | **-23.524** | **-8.274** | **-18.719** |
| | **SD** | **0.000** | **12.322** | **18.580** | **9.469** | **4.234** | **10.621** | **9.449** | **15.772** | **4.456** |
| | | | | | | | | | | |
| Metformin (450) | 301 | 0.000 | 4.630 | -9.259 | -15.741 | -25.000 | -47.222 | -48.148 | -50.926 | -35.185 |

(continued)

| | Rat | 0 | 15 | 30 | 45 | 60 | 120 | 180 | 240 | 300 |
|---|---|---|---|---|---|---|---|---|---|---|
| | 302 | 0.000 | -2.273 | -3.409 | -12.500 | -15.909 | -27.273 | -39.773 | -36.364 | -34.091 |
| | 303 | 0.000 | 2.128 | 2.128 | -5.319 | -10.638 | -23.404 | -27.660 | -30.851 | -27.660 |
| | 304 | 0.000 | 2.041 | -2.041 | -16.327 | -14.286 | -41.837 | -35.714 | -44.898 | -58.163 |
| | 305 | 0.000 | 3.738 | -3.738 | -18.692 | -22.430 | -35.514 | -42.056 | -41.121 | -29.907 |
| | **Mean** | **0.000** | **2.053** | **-3.264** | **-13.716** | **-17.653** | **-35.050** | **-38.670** | **-40.832** | **-37.001** |
| | **SD** | **0.000** | **2.655** | **4.085** | **5.188** | **5.924** | **9.881** | **7.624** | **7.715** | **12.219** |
| | | | | | | | | | | |
| Ins (0.25) + 4-CNAB (200) + Metformin (450) | 401 | 0.000 | -7.071 | -21.212 | -18.182 | -32.323 | -37.374 | -45.455 | -60.606 | -37.374 |
| | 402 | 0.000 | -6.977 | -13.953 | -25.581 | -31.395 | -24.419 | -20.930 | -26.744 | -24.419 |
| | 403 | 0.000 | -10.345 | -21.839 | -25.287 | -24.138 | -37.931 | -28.736 | -27.586 | -24.138 |
| | 404 | 0.000 | -16.822 | -30.841 | -32.710 | -37.383 | -52.336 | -47.664 | -44.860 | -37.383 |
| | 405 | 0.000 | -2.353 | -29.412 | -38.824 | -34.118 | -36.471 | -23.529 | -25.882 | -22.353 |
| | **Mean** | **0.000** | **-8.714** | **-23.452** | **-28.117** | **-31.871** | **-37.706** | **-33.263** | **-37.136** | **-29.133** |
| | **SD** | **0.000** | **5.352** | **6.855** | **7.888** | **4.890** | **9.903** | **12.483** | **15.300** | **7.568** |
| | | | | | | | | | | |
| Ins (0.1) + 4-CNAB (200) + Metformin (450) | 501 | 0.000 | 5.063 | -17.722 | -22.785 | -17.722 | -25.316 | -22.785 | -35.443 | -34.177 |
| | 502 | 0.000 | 5.263 | -1.316 | -14.474 | -23.684 | -30.263 | -40.789 | -34.211 | -47.368 |
| | 503 | 0.000 | -1.961 | -20.588 | -26.471 | -32.353 | -36.275 | -45.098 | -40.196 | -48.039 |
| | 504 | 0.000 | -14.815 | -30.864 | -34.568 | -37.037 | -28.395 | -32.099 | -30.864 | -43.210 |
| | 505 | 0.000 | 17.442 | 3.488 | -6.977 | -12.791 | -13.953 | -16.279 | -24.419 | -27.907 |
| | **Mean** | **0.000** | **2.199** | **-13.400** | **-21.055** | **-24.717** | **-26.841** | **-31.410** | **-33.026** | **-40.140** |
| | **SD** | **0.000** | **11.797** | **14.200** | **10.682** | **10.029** | **8.239** | **12.033** | **5.861** | **8.793** |

Table 2 demonstrates the same test conditions in the same rats, but quantitatively measures the percent change in blood glucose from baseline. These trends are similar to those found in Table 1. Group 1 reached a maximum decline in blood glucose from baseline of -37.156% (S.D. 8.58) at 30 minutes, rapidly rising and leveling off as shown in Table 1. Group 2 had a gradual onset of blood glucose decline from baseline at about 30 to 45 minutes, peaking at about 45 minutes, with a -40.832% (S.D. 7.715) change and leveling off. Groups 3 and 4 also had rapid onsets of action, but the decline in blood glucose from baseline caused by the lower dose of insulin peaked by about 120 minutes (at -37.706% S.D. 9.903) and the decline in blood glucose from baseline caused by the higher dose of insulin continued decreasing the glucose levels at 300 minutes, and was more effective in doing so at that time point than any of the other groups. The mean percent change at 300 minutes of Group 3 was -29.133% (S.D. 7.5688) versus Group 4 which was -40.140% (S.D. 8.793). These results are demonstrated visually in Figure 2.

**Table 3: Percent Change in Glucose Compared to Carrier Alone**

| | **Rat** | **0** | **15** | **30** | **45** | **60** | **120** | **180** | **240** | **300** |
|---|---|---|---|---|---|---|---|---|---|---|
| Ins (0.25) + 4-CNAB (200) | | | | | | | | | | |
| | 201 | -21.875 | -44.681 | -63.883 | -51.422 | -28.750 | -8.430 | -16.422 | 7.038 | -6.752 |
| | 202 | -8.654 | -51.064 | -57.111 | -34.834 | -15.000 | -11.337 | -9.091 | 1.173 | -3.537 |
| | 203 | -2.644 | -22.340 | -37.923 | -34.834 | -15.000 | -17.151 | -22.287 | 4.106 | 2.894 |
| | 204 | -6.250 | -29.787 | -52.596 | -43.128 | -22.500 | -11.337 | -22.287 | -20.821 | -0.322 |
| | 205 | -11.058 | -28.723 | -22.122 | -25.355 | -17.500 | -22.965 | -13.490 | 7.038 | -5.145 |
| | **Mean** | **-10.096** | **-35.319** | **-46.727** | **-37.915** | **-19.750** | **-14.244** | **-16.716** | **-0.293** | **-2.572** |
| | **SD** | **7.281** | **12.022** | **16.729** | **9.828** | **5.890** | **5.814** | **5.717** | **11.730** | **3.872** |
| Metformin (450) | | | | | | | | | | |
| | 301 | 29.808 | | | | | | | | |
| | 302 | 5.769 | -8.511 | -4.063 | -8.768 | -7.500 | -6.977 | -22.287 | -17.889 | -6.752 |
| | 303 | 12.981 | 2.128 | 8.352 | 5.450 | 5.000 | 4.651 | -0.293 | -4.692 | 9.325 |
| | 304 | 17.788 | 6.383 | 8.352 | -2.844 | 5.000 | -17.151 | -7.625 | -20.821 | -34.084 |
| | 305 | 28.606 | 18.085 | 16.253 | 3.081 | 3.750 | 0.291 | -9.091 | -7.625 | 20.579 |
| | **Mean** | **18.990** | **4.521** | **7.223** | **-0.770** | **1.563** | **-4.797** | **-9.824** | **-12.757** | **-2.733** |
| | **SD** | **10.269** | **11.000** | **8.396** | **6.371** | **6.070** | **9.531** | **9.157** | **7.805** | **23.720** |

(continued)

| | | | | | | | | | | |
|---|---|---|---|---|---|---|---|---|---|---|
| Ins (0.25) + 4-CNAB (200) + Metformin (450) | | | | | | | | | | |
| | 401 | 18.990 | -2.128 | -11.964 | -4.028 | -16.250 | -9.884 | -20.821 | -42.815 | -0.322 |
| | 402 | 3.365 | -14.894 | -16.479 | -24.171 | -26.250 | -5.523 | -0.293 | -7.625 | 4.502 |
| | 403 | 4.567 | -17.021 | -23.251 | -22.986 | -17.500 | -21.512 | -9.091 | -7.625 | 6.109 |
| | 404 | 28.606 | -5.319 | -16.479 | -14.692 | -16.250 | -25.872 | -17.889 | -13.490 | 7.717 |
| | 405 | 2.163 | -11.702 | -32.280 | -38.389 | -30.000 | -21.512 | -4.692 | -7.625 | 6.109 |
| | **Mean** | **11.538** | **-10.213** | **-20.090** | **-20.853** | **-21.250** | **-16.860** | **-10.557** | **-15.836** | **4.823** |
| | **SD** | **11.727** | **6.321** | **7.917** | **12.689** | **6.435** | **8.685** | **8.675** | **15.294** | **3.093** |
| Ins (0.1) + 4-CNAB (200) + Metformin (450) | | | | | | | | | | |
| | 501 | -5.048 | -11.702 | -26.637 | -27.725 | -18.750 | -14.244 | -10.557 | -25.220 | -16.399 |
| | 502 | -8.654 | -14.894 | -15.350 | -22.986 | -27.500 | -22.965 | -34.018 | -26.686 | -35.691 |
| | 503 | 22.596 | 6.383 | -8.578 | -11.137 | -13.750 | -5.523 | -17.889 | -10.557 | -14.791 |
| | 504 | -2.644 | -26.596 | -36.795 | -37.204 | -36.250 | -15.698 | -19.355 | -17.889 | -26.045 |
| | 505 | 3.365 | 7.447 | 0.451 | -5.213 | -6.250 | 7.558 | 5.572 | -4.692 | -0.322 |
| | **Mean** | **1.923** | **-7.872** | **-17.381** | **-20.853** | **-20.500** | **-10.174** | **-15.249** | **-17.009** | **-18.650** |
| | **SD** | **12.357** | **14.598** | **14.681** | **12.827** | **11.713** | **11.691** | **14.419** | **9.423** | **13.238** |

Table 3 demonstrates the percent change in blood glucose with respect to the vehicle (carrier) alone group, and produced similar results to the previous tables, and is demonstrated visually in Figure 3.

Thus, the oral formulations that were tested demonstrated a rapid onset of hypoglycemic action within 30 minutes of oral administration. The hypoglycemic effect lasted for at least 4 hours when testing was stopped. However it is postulated that glycemic control may last beyond the time tested. In addition, the composition provided a decrease in whole blood glucose concentration, starting at about 20 minutes, with a maximum decrease at about 180 minutes after oral administration. This glucose-lowering effect lasted for at least about 5 hours after the initial dose.

While certain preferred and alternative embodiments of the invention have been set forth for purposes of disclosing the invention, modifications to the disclosed embodiments may occur to those who are skilled in the art. Accordingly, the appended claims are intended to cover all embodiments of the invention and modifications thereof that do not depart from the spirit and scope of the invention.

## Claims

1. An oral dosage form comprising a therapeutically effective dose of insulin and a biguanide, further comprising a pharmaceutically acceptable delivery agent that facilitates absorption of insulin from the gastrointestinal tract.

2. The dosage form according to claim 1, wherein the biguanide is metformin, prefera-blymetformin hydrochloride.

3. The dosage form according to any one of claims 1 to 2, wherein the biguanide comprises from about 500 mg to about 850 mg of metformin, preferably from about 90 mg to about 3000 mg of metformin

4. The dosage form according to any one of claims 1 to 3, wherein said insulin comprises a dose of unmodified insulin that achieves a comparable reduction in blood glucose concentration in mammals compared to a subcutaneous insulin injection in those mammals.

5. The dosage form according to any one of claims 1 to 4, wherein said insulin provides a lower concentration of insulin in the peripheral blood circulation under acute, sub-acute or chronic conditions as compared to the peripheral blood insulin concentration obtained via the subcutaneous injection.

6. The dosage form according to any of claims 1 to 5, wherein the amount of insulin contained in said dosage form is from about 10 Units to about 600 Units, preferably from about 200 Units to about 350 Units.

7. The dosage form according to claim 1, wherein said delivery agent is of the following formula or a pharmaceutically acceptable salt thereof,

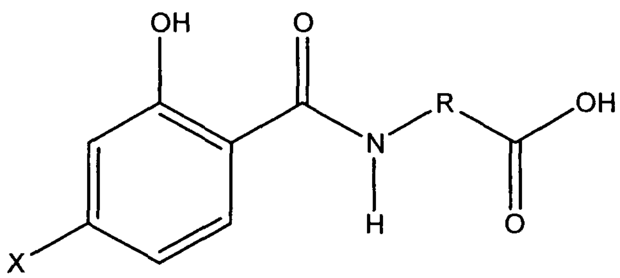

wherein

X is hydrogen or halogen;
R is substituted or unsubstituted C1-C3 alkylene, substituted or unsubstituted C1-C3 alkenylene, substituted or unsubstituted C1-C3 alkyl (arylene), substituted or unsubstituted C1-C3 aryl (alkylene), wherein, preferably, X is a halogen, more preferably chlorine.

8. The dosage form according to claim 7, wherein R is $C_3$ alkylene.

9. The dosage form according to any of claims 7 to 8, wherein said delivery agent is 4-[(4-chloro, 2-hydroxybenzoyl) amino]butanoic acid.

10. The dosage form according to any of the foregoing claims, wherein the amount of delivery agent contained in said tablet is from about 20 mg to about 600 mg, preferably from about 150 mg to about 400 mg.

11. The dosage form according to any of the preceding claims, wherein the oral dosage form is in the form of a solid, preferably a tablet or capsule.

12. The oral dosage form according to any of claims 1-11 for use in a method for treating diabetes and conditions associated with diabetes in a mammal, comprising orally administering to the mammal said oral dosage form, , wherein, preferably,said oral dosage form is administered on a chronic basis, wherein, more preferably,the biguanide is metformin, even more preferably, metformin hydrochloride.

**13.** The oral dosage form according to claim 12, wherein the biguanide administered is from about 500 mg to about 850 mg of metformin, preferably from about 10 Units to about 600 Units, more preferably from about 200 Units to about 350 Units.

**14.** The oral dosage form according to claim 12, wherein the amount of insulin is 0.25 mg to about 1.5 mg.

**15.** The oral dosage form according to claim 12, wherein said diabetes is impaired glucose tolerance, early stage diabetes, late stage diabetes, non-insulin dependent diabetes or insulin dependent diabetes.

**16.** The oral dosage form according to claim 12, wherein said mammal is a human.

**17.** The oral dosage form according to claim 12, wherein said oral dosage form does not induce weight gain.

**18.** The oral dosage form according to any of claims 1-11 for use in a method of treating diabetic mammal patients, said method comprising:

orally administering to a mammal on a chronic basis said oral dosage form according to any of claims 1 - 11, discontinuing said chronic administration, and
obtaining, as a result of said chronic administration, an improved effect as compared to baseline levels before said chronic administration, wherein, preferably, said improved effect is selected from the group consisting of

(a) improved glucose tolerance;
(b) improved glycemic control;
(c) improved glucose homeostasis;
(d) spared β-cell function;
(e) prevention of β-cell death;
(f) prevention of β-cell dysfunction;
(g) reduction in systemic hyperinsulinemia;
(h) delay in the onset of overt or insulin dependent diabetes;
(i) reduction in the incidence of a disease state associated with chronic dosing of insulin;
(j) improved insulin utilization and insulin sensitivity;
(k) improved insulin secretion capacity; or
(l) or any combination thereof.

**19.** The oral dosage form according to claim 18, wherein said improved effect is improved glucose tolerance, further comprising the step of achieving said improved glucose tolerance without any statistically significant weight gain by said patient over said period of chronic administration, orwithout any statistically significant risk of hypoglycemia in said mammal over said period of chronic administration,without any statistically significant risk of hyperinsulinemia in said mammal over said period of chronic administration.

**20.** The oral dosage form according to claims 18-19, wherein said insulin comprises a dose of unmodified insulin that achieves a comparable reduction in blood glucose concentration in mammals compared to a subcutaneous insulin injection in those mammals, or wherein said insulin provides a lower concentration of insulin in the peripheral blood circulation under acute, sub-acute or chronic conditions as compared to the peripheral blood insulin concentration obtained via the subcutaneous injection.

**21.** The dosage form according to any of claims 1-11 in which there is a lowering of serum blood glucose starting at about 15 minutes after oral administration and a sustained effect lasting about 5 hours, or in which there is an average decrease in blood glucose by about 37% to about 40% at about 30 minutes to about 300 minutes after administration.

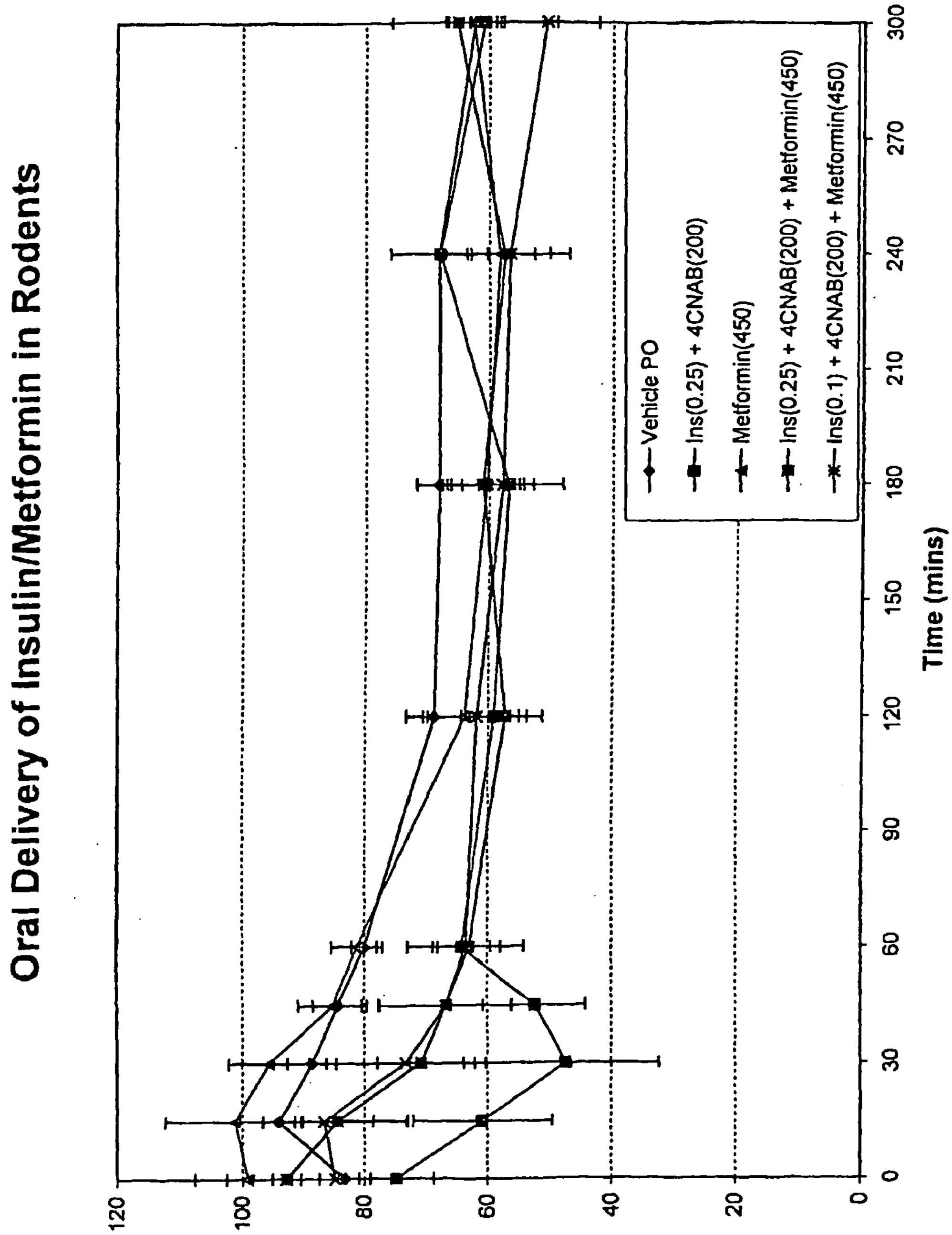
Oral Delivery of Insulin/Metformin in Rodents
Whole Blood Glucose (mg/dL)
0
20
40
60
80
100
120
Time (mins)
0
30
60
90
120
150
180
210
240
270
300
Vehicle PO
Ins(0.25) + 4CNAB(200)
Metformin(450)
Ins(0.25) + 4CNAB(200) + Metformin(450)
Ins(0.1) + 4CNAB(200) + Metformin(450)

FIGURE 1

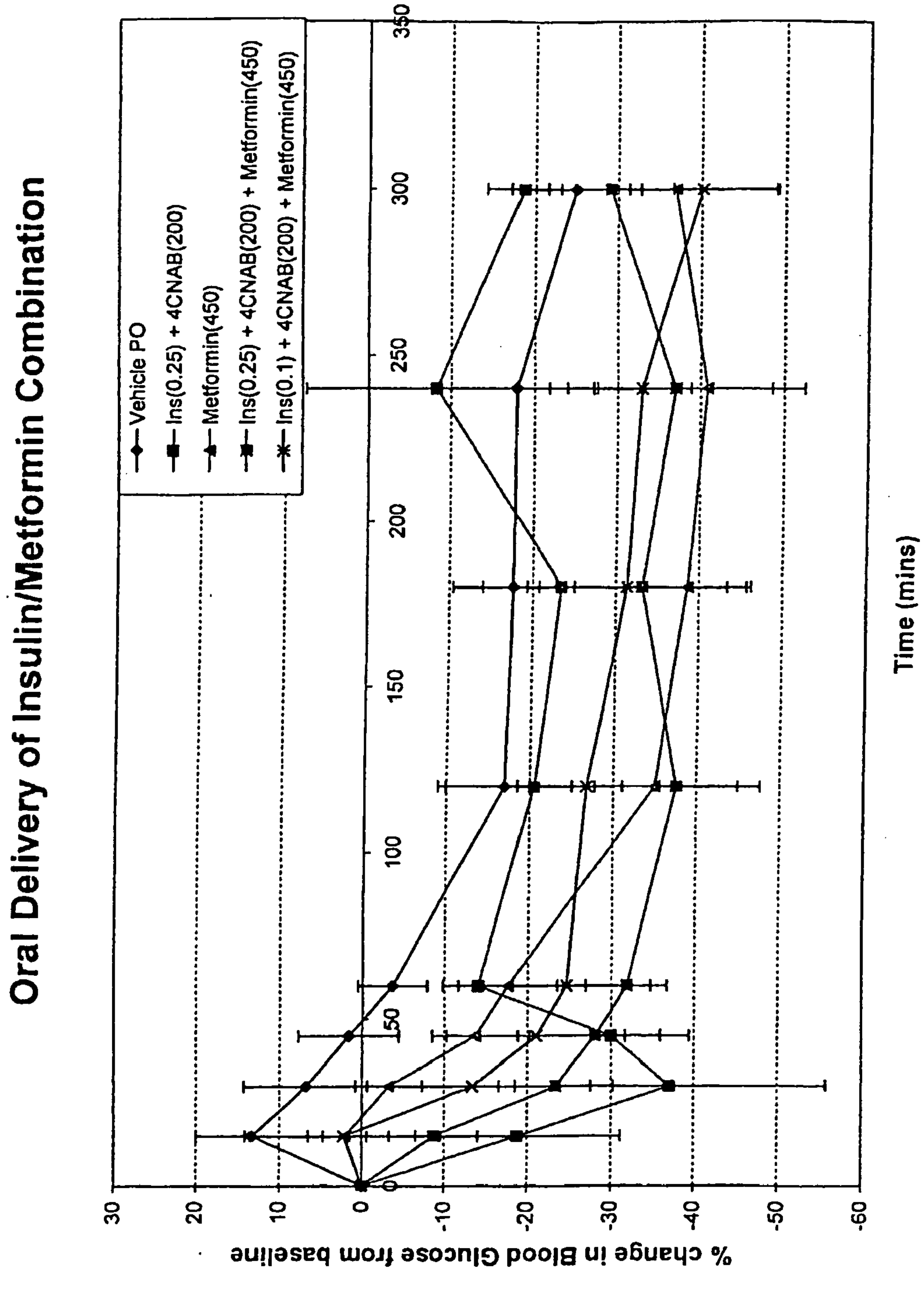
Oral Delivery of Insulin/Metformin Combination
Vehicle PO
Ins(0.25) + 4CNAB(200)
Metformin(450)
Ins(0.25) + 4CNAB(200) + Metformin(450)
Ins(0.1) + 4CNAB(200) + Metformin(450)
% change in Blood Glucose from baseline
30
20
10
0
-10
-20
-30
-40
-50
-60
0
50
100
150
200
250
300
350
Time (mins)

FIGURE 2

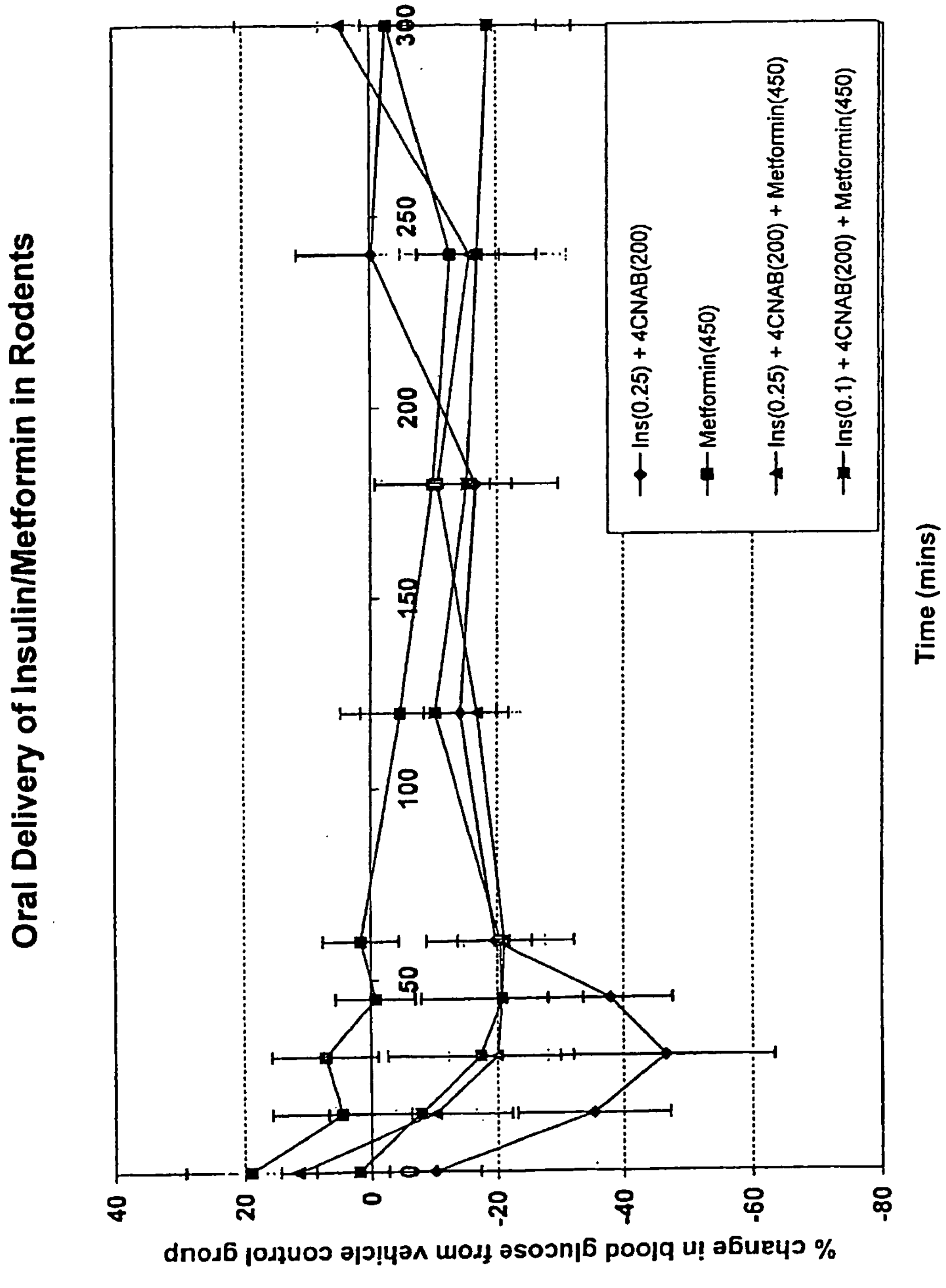
Oral Delivery of Insulin/Metformin in Rodents
% change in blood glucose from vehicle control group
40
20
0
-20
-40
-60
-80
0
50
100
150
200
250
300
Time (mins)
Ins(0.25) + 4CNAB(200)
Metformin(450)
Ins(0.25) + 4CNAB(200) + Metformin(450)
Ins(0.1) + 4CNAB(200) + Metformin(450)

FIGURE 3

Europäisches Patentamt
European Patent Office
Office européen des brevets

# EUROPEAN SEARCH REPORT

Application Number
EP 10 00 6250

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/062587 A2 (EMISPHERE TECH INC [US]; GOLDBERG MICHAEL [US]; ARBIT EHUD [US]) 29 July 2004 (2004-07-29)<br>* the whole document *<br>----- | 1-21 | INV.<br>A61K38/28 |
| X | WO 03/057170 A2 (EMISPHERE TECH INC [US]; ARBIT EHUD [US]; ABBAS RICHAT [US]; GOLDBERG) 17 July 2003 (2003-07-17)<br>* the whole document *<br>----- | 1-21 | |
| Y | WO 03/057650 A2 (EMISPHERE TECH INC [US]; BHANDARKAR SATEJ [US]; MAJURU SHINGAI [US]; L) 17 July 2003 (2003-07-17)<br>* the whole document *<br>----- | 1-21 | |
| Y | HERMANN L S: "Combination therapy with insulin and metformin"<br>ENDOCRINE PRACTICE, AMERICAN ASSOCIATION OF CLINICAL ENDOCRINOLOGY, US,<br>vol. 4, no. 6,<br>1 November 1998 (1998-11-01), pages 404-412, XP009116875<br>ISSN: 1530-891X<br>* see the whole document *<br>----- | 1-21 | TECHNICAL FIELDS SEARCHED (IPC)<br>A61K |
| Y | MODI PANKAJ ET AL: "The evolving role of oral insulin in the treatment of diabetes using a novel RapidMist(TM) System"<br>DIABETES-METABOLISM RESEARCH AND REVIEWS,<br>vol. 18, no. Suppl. 1,<br>January 2002 (2002-01), pages S38-S42, XP002527898<br>ISSN: 1520-7552<br>* see especially p. 539, right col. last para. *<br>----- | 1-21 | |

The present search report has been drawn up for all claims

1

| Place of search | Date of completion of the search | Examiner |
|---|---|---|
| Munich | 10 August 2010 | Grosskopf, Ruediger |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 00 6250

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2010

| Patent document cited in search report | Publication date | Patent family member(s) | Publication date |
|---|---|---|---|
| WO 2004062587 A2 | 29-07-2004 | AU 2004204727 A1 | 29-07-2004 |
| | | CA 2511530 A1 | 29-07-2004 |
| | | EP 1592438 A2 | 09-11-2005 |
| | | JP 2006515620 T | 01-06-2006 |
| | | NZ 541058 A | 30-06-2008 |
| WO 03057170 A2 | 17-07-2003 | AU 2003226436 A1 | 24-07-2003 |
| | | CA 2471769 A1 | 17-07-2003 |
| | | EP 1469812 A2 | 27-10-2004 |
| | | JP 2005525308 T | 25-08-2005 |
| | | US 2003198666 A1 | 23-10-2003 |
| | | US 2006234913 A1 | 19-10-2006 |
| WO 03057650 A2 | 17-07-2003 | AU 2003223158 A1 | 24-07-2003 |
| | | CA 2471144 A1 | 17-07-2003 |
| | | EP 1469827 A2 | 27-10-2004 |
| | | JP 2005520803 T | 14-07-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 237138 A
- US 60346746 A
- US 60347312 A
- US 60368617 A
- US 60374979 A
- US 60389364 A
- US 60438195 A
- US 60438451 A
- US 60578967 A
- US 60452660 A
- US 60488465 A
- US 60518168 A
- US 60535091 A
- US 60540462 A
- WO 03057170 A
- WO 03057650 A
- WO 0202509 A
- US 0400273 W
- US 6309633 B
- US 5359030 A
- US 5438040 A
- US 5681811 A
- US 4421685 A
- US 5474978 A
- US 5534488 A
- US 5650386 A
- US 5773647 A
- US 5776888 A
- US 5804688 A
- US 5866536 A
- US 5876710 A
- US 5879681 A
- US 5939381 A
- US 5955503 A
- US 5965121 A
- US 5989539 A
- US 5990166 A
- US 6001347 A
- US 6051561 A
- US 6060513 A
- US 6090958 A
- US 6100298 A
- US 5766633 A
- US 5643957 A
- US 5863944 A
- US 6071510 A
- US 6358504 B
- WO 0134114 A
- WO 0121073 A
- WO 0141985 A
- WO 0132130 A
- WO 0132596 A
- WO 0144199 A
- WO 0151454 A
- WO 0125704 A
- WO 0125679 A
- WO 0050386 A
- WO 0047188 A
- WO 0007979 A
- WO 0006534 A
- WO 9825589 A
- WO 0219969 A
- WO 0059863 A
- WO 9528838 A
- WO 0220466 A
- WO 02069937 A
- WO 02070438 A
- WO 9630036 A
- WO 9736480 A
- WO 9834632 A
- WO 0046182 A
- US 0406943 W


### Non-patent literature cited in the description


- **Yki-Jarvinen H et al.** *Ann Intern Med,* 1999, vol. 130, 389-396
- **Yki-Jarvinen H et al.** *N Engl J Med,* 1992, vol. 327, 1426-1433
- **Lindstrom T et al.** *Diabet Med,* 1999, vol. 16, 820-826
- **UKPDS.** *Lancet,* 1998, vol. 353, 837-853
- **Yki-Jarvinen H.** *Ann Intern Med,* 1999, vol. 131, 182-188
- **Avilés-Santa L.** *Ann Intern Med,* 1999, vol. 131, 182-188
- **Strowig SM ; Aviles-Santa ML ; Raskin P.** *Diabetes Care,* July 2004, vol. 27 (7), 1577-83
- Remington's Pharmaceutical Sciences. Mack Publishing Co,